# EUROPÄISCHE PATENTSCHRIFT

(11) **EP 1 531 768 B1**
(45) Veröffentlichungstag und Bekanntmachung des Hinweises auf die Patenterteilung: **26.04.2006**
(21) Anmeldenummer: 03790816.7
(22) Anmeldetag: 25.07.2003
(51) Int. Cl.: A61F 5/00

(54) **ORTHOP DISCHE VORRICHTUNG ZUR KORREKTUR VON ZEHENFEHLSTELLUN GEN**
ORTHOPAEDIC DEVICE FOR CORRECTING ABNORMAL POSITIONS OF THE TOES
DISPOSITIF ORTHOPEDIQUE POUR CORRIGER DES MALPOSITIONS DES ORTEILS

(30) Priorität: 30.08.2002 DE 10240121
(43) Veröffentlichungstag der Anmeldung: 25.05.2005
(73) Patentinhaber: Huber, Vitus Maria, 80637 München (DE); Krauss, Axel, 83666 Waakirchen (DE)
(72) Erfinder: Huber, Vitus Maria, 80637 München (DE); Krauss, Axel, 83666 Waakirchen (DE)
(74) Vertreter: nospat Patentanwälte
(86) Internationale Anmeldenummer: PCT/EP2003/008204
(87) Internationale Veröffentlichungsnummer: WO 2004/019835

(56) Entgegenhaltungen:
- DE-A- 10 034 354
- DE-U- 1 881 215
- DE-U- 8 902 545
- US-A- 1 183 062

## Beschreibung

Die Erfindung betrifft eine orthopädische Vorrichtung zur Korrektur von Zehenfehlstellungen nach dem Oberbegriff des Anspruches 1.

Aus der DE 100 34 354 A1 ist eine Fußbandage zur Behandlung von Fehlstellungen des Großzehs, beispielsweise von Hallux valgus, bekannt. Sie weist eine in Fußlängsrichtung dehnbare Aufnahmeeinrichtung für den Großzeh auf, welche mit einem freien Ende an einem im Bereich des Mittelfußes angeordnete, diesen umgebende Ringbandage angebunden ist, sodass auf den Großzeh eine redressierende Kraft in Richtung der anatomischen Sollstellung des Zehs wirkt.

Solche Vorrichtungen werden erfahrungsgemäß nur ungern und unzuverlässig vom Patienten angelegt, weil sie stark auftragen und im normalen Schuhwerk als hinderlich oder nach längerer Tragedauer sogar lästig oder schmerzhaft empfunden werden. Hierdurch ist der Behandlungserfolg bei Anwendung einer derartigen Bandage nicht sichergestellt.

Aus der DE 1 881 215 U1 ist eine Ballenschiene bekannt, welche als Biegefeder wirkend entlang der Fußinnenseite verläuft und an ihrem zehenseitigen Ende eine Ringöse aufweist, welche zur Aufnahme des Großzehs dient. Am gegenüberliegenden Ende weist die Ballenschiene eine Umbiegung auf, welche sich um die Ferse legen lässt. Hierdurch lässt sich eine Großzehe aus einer eingebogenen Zehenfehlstellung in die Normalstellung verbringen. Diese Schiene weist erhebliche Nachteile auf, z. B. wird sie von den Trägern als extrem unbequem im Tragekomfort empfunden, sodass diese nur sehr ungern angelegt wird, was den Behandlungserfolg nicht gewährleistet.

Aus dem deutschen Gebrauchsmuster DE 8 902 545.8 U1 ist eine Vorrichtung zur Behandlung von Großzehen bekannt, welche einen Strumpf mit einem den Großzehen umgebenden Großzehenfach aufweist und eine entlang der Fußinnenseite verlaufende. Schiene aufweist, welche in einer auf den Socken aufgenähten Tasche angeordnet ist. Eine derartige Vorrichtung zur Behandlung von Großzehen ist zur Behandlung in der Nacht- bzw. Schlafzeit gedacht. Nachteilig ist, dass die Bewegungsfreiheit der geschienten Großzehe in der Flexion-Extension-Bewegungsrichtung des Großzehs unterbunden ist. Diese Vorrichtung eignet sich somit nicht für eine Dauerbehandlung. Ein Tragen dieser Vorrichtung in einem Schuh ist für einen Patienten sehr unangenehm und schränkt die Bewegungsfreiheit stark ein.

Weiterhin sind Spreizvorrichtungen bekannt, die als Keil ausgebildet sind und im Zwischenraum zwischen dem Großzeh und dem zweiten Zeh angeordnet sind, sodass der Großzeh zur Fußinnenseite hin gedrückt wird. Diese Vorrichtungen haben den Nachteil, dass sie sich zur Kraftausübung an den benachbarten Zehen abstützen und somit eine Fehlstellung der Nachbarzehen verursachen oder fördern können.

Aus der US 1,183,062 ist eine Gelenkschiene zur Korrektur von Hallux-Valgus-Fehlstellungen bekannt, bei der ein erster Gelenkschenkel im Bereich des Mittelfußes befestigt ist und ein zweiter Gelenkschenkel im Bereich des Großzehen befestigt ist, wobei die Gelenkschenkel unterhalb des Patientenfußes gelenkig miteinander verbunden sind. Durch den Verlauf der Gelenkschiene entlang der Fußunterseite findet ein ständiges Auftreten des Patienten auf der Gelenkschiene statt.

Aufgabe der Erfindung ist es, eine Vorrichtung zu schaffen, mit der Valgus-Fehlstellungen von Zehen, d. h. Fehlstellungen von einer oder mehreren Zehen zur Fußaußenseite hin, behandelt werden können. Weiterhin soll die Vorrichtung angenehm tragbar, insbesondere ohne nennenswerte Beeinträchtigung im Alltag tragbar sein. Der Behandlungserfolg soll gegenüber dem Stand der Technik verbessert werden.

Insbesondere ist es Aufgabe der Erfindung, eine gelenkige orthopädische Vorrichtung mit kompaktem Aufbau des Gelenks und einem verbesserten Tragekomfort zur Vermeidung von Scheuerungen, Blasen und Wundlaufen des Patienten bereitzustellen.

Diese Aufgabe wird mit einer orthopädischen Vorrichtung zur Korrektur von Zehenfehlstellungen mit den Merkmalen des Anspruchs 1 gelöst. Vorteilhafte Weiterbildungen der Erfindung sind in den Unteransprüchen angegeben.

Im Folgenden wird die Erfindung anhand der Zeichnungen beispielhaft näher erläutert. Es zeigen:
- Fig. 1: schematisch eine Unteransicht auf eine erste erfindungsgemäße Vorrichtung;
- Fig. 1a: schematisch eine Unteransicht auf eine zweite besonders bevorzugte Ausführungsform der erfindungsgemäßen Vorrichtung;
- Fig. 2: schematisch die erfindungsgemäße Vorrichtung gem. Fig. 1 in einer Ansicht auf die Fußinnenseite;

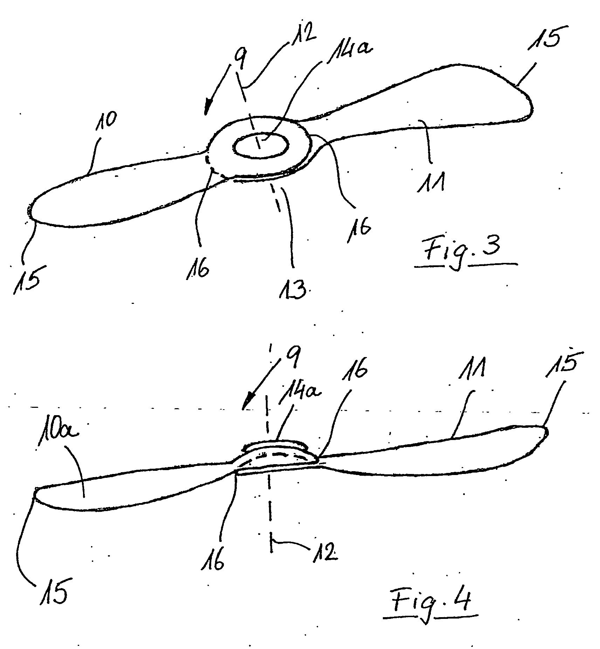

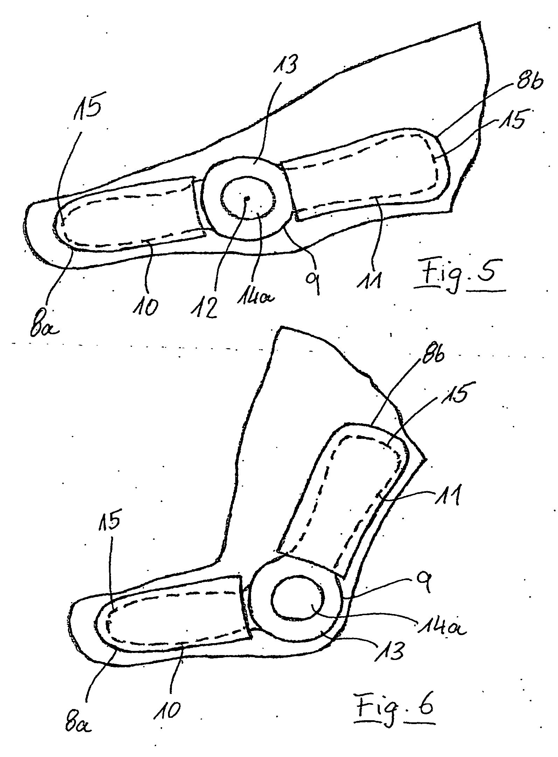

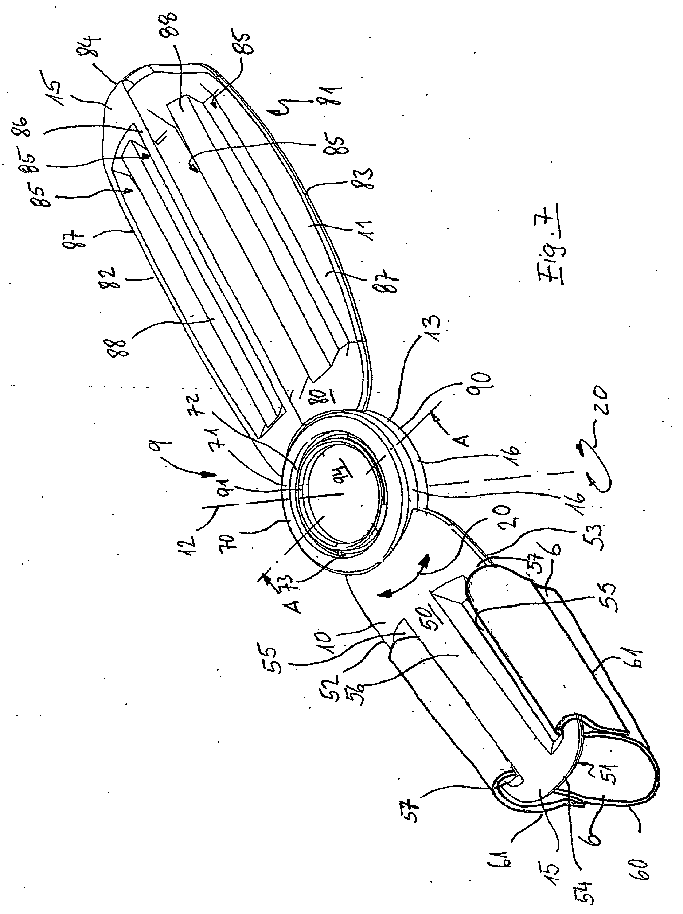

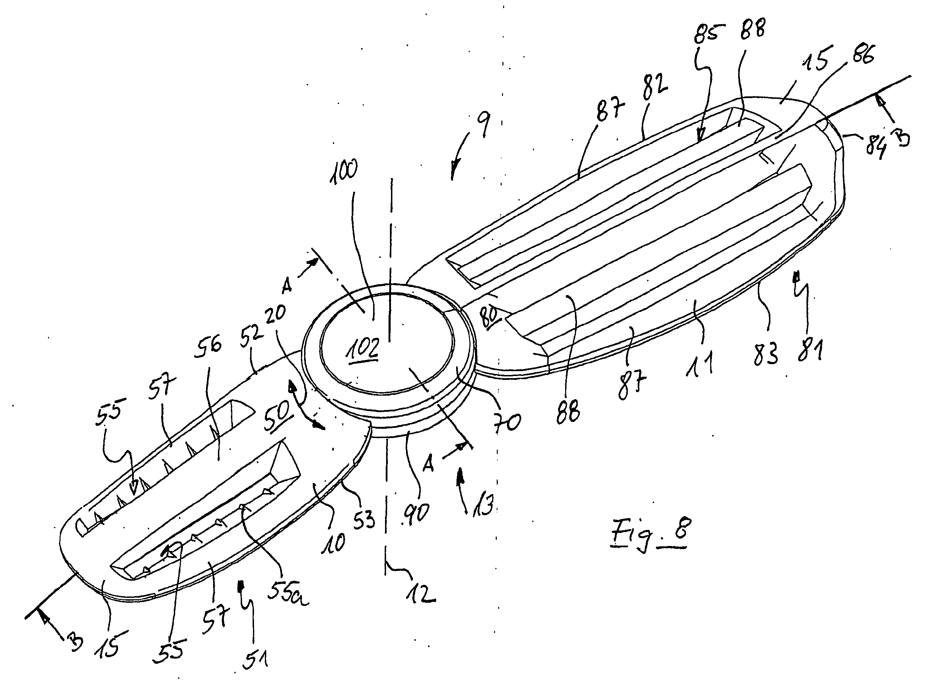

- Fig. 3: schematisch eine Gelenkschiene der erfindungsgemäßen Vorrichtung in einer perspektivischen Detailansicht;

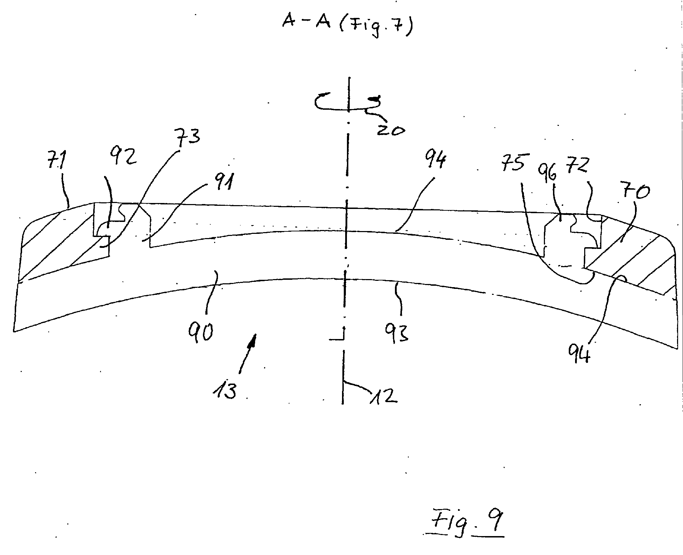

- Fig. 4: schematisch die Gelenkschiene aus Fig. 3 in einer Draufsicht;
- Fig. 5: schematisch eine Ausschnittsansicht auf einen Vorderfußbereich (Zehe und Mittelfuß) einer dritten Ausfüh- rungsform einer erfindungsgemäßen Vorrichtung in einer gestreckten Stellung;
- Fig. 6: schematisch eine Ausschnittsansicht auf einen Vorderfußbereich (Zehe und Mittelfuß) der dritten Ausführungsform einer erfindungsgemäßen Vorrichtung in einer abgewinkelten Stellung;
- Fig. 7: eine perspektivische Ansicht einer weiteren Ausführungsform der Gelenkschiene der erfindungsgemäßen Vorrichtung;
- Fig. 8: die Gelenkschiene gemäß Fig. 7 mit einer abgedeckten Gelenkeinrichtung;
- Fig. 9: einen Querschnitt durch die Gelenkeinrichtung der Gelenkschiene gemäß Fig. 7, entlang der Linie A-A in Fig. 7;
- Fig. 10: einen Querschnitt durch die Gelenkeinrichtung der Gelenkschiene gemäß Fig. 8 entlang der Linie A-A in Fig. 8;
- Fig. 11: einen Längsschnitt durch die Gelenkschiene gemäß Fig. 8 entlang der Linie B-B in Fig. 8;
- Fig. 12: eine perspektivische Darstellung einer weiteren Ausführungsform der Gelenkschiene der erfindungsgemäßen Vorrichtung;
- Fig. 13: eine perspektivische Darstellung der Gelenkschiene gemäß Fig. 12 mit einer abgedeckten Gelenkeinrichtung;
- Fig. 14: einen Querschnitt durch die Gelenkeinrichtung der Gelenkschiene gemäß Fig. 12 entlang der Linie A-A;
- Fig. 15: einen Querschnitt durch die Gelenkeinrichtung der Gelenkschiene gemäß Fig. 13 entlang der Linie A-A;
- Fig. 16: einen Längsschnitt durch die Gelenkschiene gemäß Fig. 12 entlang der Linie B-B;
- Fig. 17: einen Längsschnitt durch die Gelenkschiene gemäß Fig. 13 entlang der Linie B-B;
- Fig. 18: eine weitere Ausführungsform einer Gelenkeinrichtung der Gelenkschiene in einem teilgeschnittenen Längsschnitt;
- Fig. 19: einen Querschnitt durch einen Gelenkschienenschenkel und eine zugehörige Ringbandage im Bereich des Großzehs;
- Fig. 20: einen Querschnitt durch einen Gelenkschienenschenkel und eine Ringbandage der erfindungsgemäßen Vorrichtung im Bereich des Mittelfußes;
- Fig. 21: einen zweiten Querschnitt durch einen Gelenkschienenschenkel und die zugehörige Ringbandage der erfindungsgemäßen Vorrichtung im Bereich des Mittelfußes.

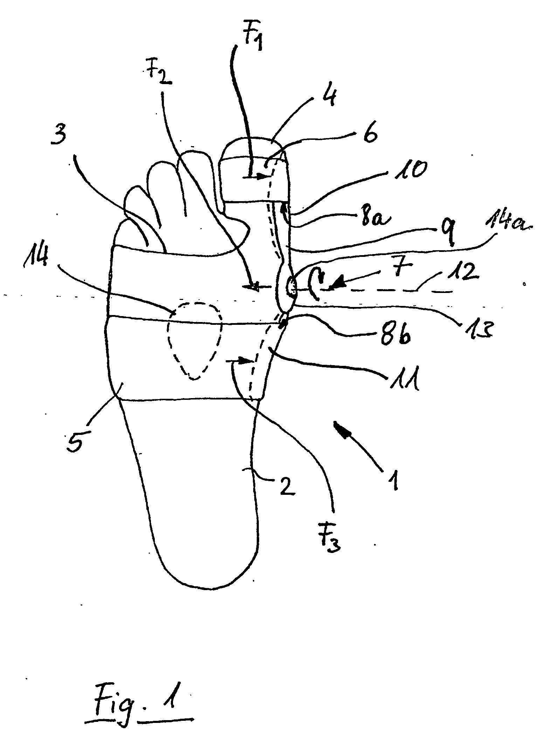

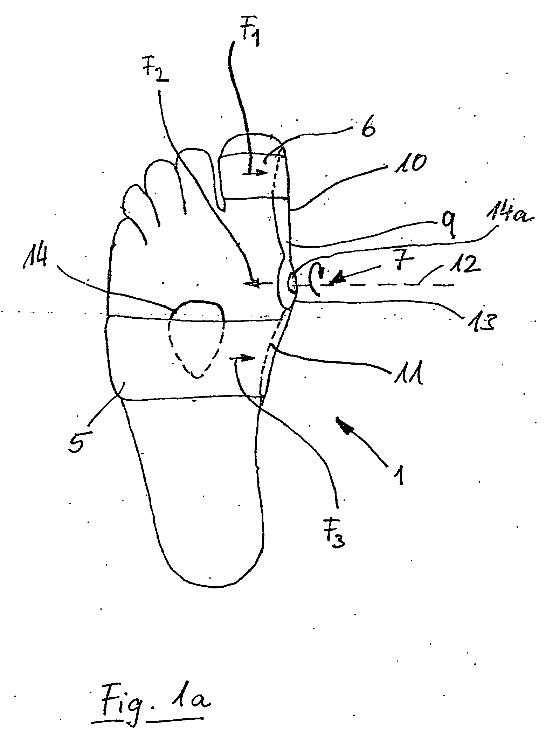

Eine erste Ausführungsform der erfindungsgemäßen Vorrichtung 1 (Fig. 1) weist einen im Zehenbereich offenen Strumpf 2 oder dergleichen Umhüllungselement für den Fuß auf. Im Bereich der Zehen weist der Strumpf 2 eine Öffnung auf, welche durch eine Begrenzungskante 3 begrenzt ist. Weiterhin weist die erfindungsgemäße Vorrichtung 1 eine Großzehenaufnahme, z.B. ein Großzehenfach 4 auf, welches mit dem Strumpf 2 einstückig verbunden oder an diesen Strumpf 2 angesetzt ist. Das Großzehenfach 4 umgibt den Großzeh umfänglich vorzugsweise vollständig und ist im Bereich des freien Zehenendes offen ausgestaltet. Bevorzugt ist das Großzehenfach 4 jedoch vorne geschlossen ausgebildet, da dies einen besseren Schutz gegen Verrutschen des Großzehenfachs 4 relativ zum Großzeh gewährleistet.

Im Bereich des Mittelfußes weist die erfindungsgemäße Vorrichtung 1 eine erste Ringbandage 5 auf, welche den Mittelfuß vorzugsweise vollständig umgibt und zweckmäßigerweise mit dem Strumpf 2 verbunden ist. Vorteilhafterweise umgibt die erste Ringbandage 5 den Strumpf 2 im Bereich des Mittelfußes außenseitig.

Das Großzehenfach 4 im Bereich des freien Endes der Großzehe umgebend ist eine zweite Ringbandage 6 angeordnet, welche den Großzeh umfänglich bevorzugt vollständig umgibt. Die Ringbandagen 5 und 6 sind vorzugsweise aus einem biegsamen, schmiegsamen, zirkulär zugstarren, d. h. in Umfangsrichtung zugstarren, Material ausgebildet, beispielsweise aus einem Gewebeband oder einem zugstarren Klebeband. Im Bereich einer Fußinnenseite 7 ist sowohl die erste Ringbandage 5 als auch die zweite Ringbandage 6 teilbereichsweise nicht mit dem Strumpf 2 bzw. dem Großzehenfach 4 verbunden, sodass zwischen den Ringbandagen 5, 6 und dem Großzehenfach 4 bzw. dem Strumpf 2 Befestigungs-/Aufnahmeeinrichtungen 8a, 8b, beispielsweise Einstecktaschen, ausgebildet sind.

Als Fußinnenseite 7 ist die Längsseite eines Fußes definiert, welche zum benachbarten Fuß weist. Die Außenseite des Fußes ist die der Innenseite gegenüberliegende Längsseite des Fußes.

Entlang der Fußinnenseite 7 erstreckt sich von der Aufnahmeeinrichtung 8b hin zur Aufnahmeeinrichtung 8a eine Biegeschiene 9. Die Biegeschiene 9 ist als Gelenkbiegeschiene ausgebildet und weist einen ersten Gelenkschienenschenkel 10 und einen zweiten Gelenkschienenschenkel 11 auf, welche um eine Schwenkachse 12 schwenkbar mittels einer Gelenkeinrichtung 13 gelenkig verbunden sind. Die Gelenkeinrichtung 13 ist bezüglich des Strumpfes 2 bzw. des Fußes derart angeordnet, dass die Schwenkachse 12 in etwa der Gelenkachse des Großzehengrundgelenks in der Flexion-Extensionsrichtung 20 , d.h. in der natürlichen Beugerichtung, also der dorsalen und plantaren Beugerichtung entspricht. Von der Gelenkeinrichtung 13 aus erstreckt sich der erste Gelenkschienenschenkel 10 bis in die Aufnahmeeinrichtung 8a. Von der Gelenkeinrichtung 13 aus erstreckt sich der zweite Gelenkschienenschenkel 11 bis in die zweite Aufnahmeeinrichtung 8b. Somit ist die Gelenkeinrichtung 13 beim Patienten in etwa im Bereich einer fußinnenseitigen, für die Hallux-valgus-Fehlstellungen typischen Ausbuchtung (Pseudoexostose) angeordnet, welche oftmals fußinnenseitig über die Sollfußumfangskontur hervorsteht. Somit wird beim Tragen einer erfindungsgemäßen Vorrichtung 1 im Bereich der Großzehe auf diese eine Kraft F1, welche in Mediolateralrichtung hin zur Fußinnenseite wirkt, ausgeübt. Im Bereich des Zehengrundgelenks wird über die Ausbuchtung eine Kraft F2 in entgegengesetzter Richtung ausgeübt. Eine aus den Kräften F1 und F2 resultierende Abstützkraft F3 wird in der Aufnahmeeinrichtung 8b von der ersten Ringbandage 5 aufgenommen.

Im Bereich der Fußsohle ist zweckmäßigerweise hinter den Grundgelenken der Zehen zur retrokapitalen Abstützung des Mittelfußes eine Pelotte 14,insbesondere eine Spreizfußpelotte eingesetzt. Diese bewirkt ein unterstützendes Aufrichten des Quergewölbes, was einen weiteren positiven Einfluss auf die Korrektur der Zehenfehlstellungen hat.

Bei der ersten Ausführungsform gemäß Fig. 1 mit den Ringbandagen 5, 6 ist auch die Verwendung eines normalen Strumpfes 2 bzw. einer normalen Strumpfstrickung möglich und zweckmäßig, da die zirkular zugstarren Ringbandagen 5, 6 eine ausreichend große Kraftübertragung gewährleisten.

In einer einfachst denkbaren besonders bevorzugten Ausführungsform (Fig. 1a) besteht die erfindungsgemäße Vorrichtung 1 lediglich aus den Ringbandagen 5, 6, der Biegeschiene 9 und ggfs. der Pelotte 14, sodass die Biegeschiene 9 zumindest teilweise direkt auf der Fußhaut des Patienten aufliegt. Diese einfachste Ausführungsform der erfindungsgemäßen Vorrichtung 1 hat sich besonders bewährt, da ein unbeabsichtigtes Verrutschen der Vorrichtung 1 relativ zum Fuß des Patienten gerade durch das Fehlen der Strumpfzwischenlage verhindert ist. Somit ist durch das Weglassen des Strumpfes überraschenderweise ein hoher Tragekomfort verwirklicht. Außerdem stellt eine Vorrichtung 1 ohne einen Strumpf die in der Herstellung kostengünstigste Ausführungsform dar. Bei der Ausführungsform gemäß Fig. 1a sind die Ringbandagen 4, 5 jeweils mit dem zugehörigen Gelenkschienenschenkel 10, 11 verbunden.

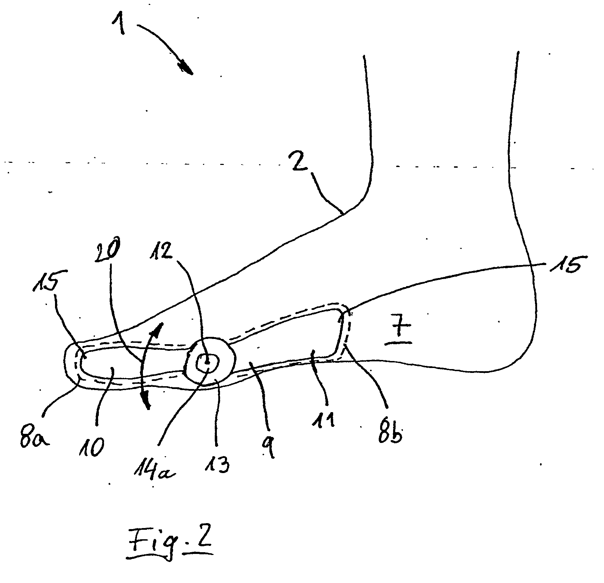

Gemäß einer dritten Ausführungsform der erfindungsgemäßen Vorrichtung (Fig. 2, 5, 6) sind die Aufnahmeeinrichtungen 8a und 8b als auf den Strumpf 2 aufgenähte oder dergleichen befestigte Taschen ausgebildet. Bei dieser Ausführungsform ist es zweckmäßig, den Strumpf 2 als so genannten Kompressionsstrumpf auszubilden, damit auch ohne die Ringbandagen 5, 6 eine ausreichend große Kraftübertragung, d. h. eine ausreichend große Einleitung der Kräfte F1 und F3 in den Fuß gewährleistet ist.

Die Gelenkeinrichtung 13 ist im Wesentlichen dreiteilig aus dem ersten Gelenkschienenschenkel 10, dem zweiten Gelenkschienenschenkel 11 und einer Gelenkschienenschenkelverbindungseinrichtung 14a, insbesondere einer Hohlniet, aufgebaut. Die Gelenkschienenschenkel 10, 11 weisen jeweils ein freies Ende 15 und ein gelenkseitiges Ende 16 auf. Die gelenkseitigen Enden 16 weisen in etwa eine Kugelkalottenraumform auf, welche zueinander derart korrespondierend ausgebildet sind, dass die jeweils gelenkseitigen Enden 16 der Gelenkschienenschenkel 10, 11 formschlüssig passend ineinanderlegbar sind. Aus den kugelkalottenförmigen gelenkseitigen Enden 16, welche mittels der Hohlniet 14a verbunden sind, ist die Gelenkeinrichtung 13 gebildet, welche durch die kugelkalottenförmige Ausgestaltung der gelenkseitigen Enden 16 eine hohe Stabilität in der Mediolateralrichtung aufweist.

Weiterhin ist durch die Ausbeulung der Gelenkeinrichtung 13 als kombinatorische Wirkung von Vorteil, dass hierdurch in einfacher Art und Weise eine individuelle Anpassung der Gelenkeinrichtung 14a an die Fußkontur des Patienten im Bereich des Großzehengrundgelenks möglich ist. Dies ist besonders vorteilhaft, da im Bereich des Großzehengrundgelenks bei Vorliegen einer Hallux-valgus-Fehlstellung oftmals ballenförmige Ausbuchtungen (d. h. eine sogenannte Pseudoexostose) am Fuß vorliegen. In besonders bevorzugter Art und Weise ist die Kalottenform, -tiefe, -größe und der Kalottendurchmesser individuell auf den Patientenfuß abgestimmt.

Die Gelenkschienenschenkel 10, 11, welche sich von der Gelenkeinrichtung 13 weg erstrecken, sind vorteilhafterweise im Querschnitt bzw. ihrer gesamten Raumform an die Fußkontur angepasst ausgebildet. Für die Ausbildung der gelenkseitigen Enden 16 der Gelenkschienenschenkel 10, 11 sind nicht nur kugelkalottenförmige Raumformen, sondern jede Art von um die Achse 12 rotationssymmetrische, zueinander korrespondierende Raumformen, insbesondere kegelstumpfförmige oder im Querschnitt parabelförmige Raumformen, geeignet.

Als Material für die Biegeschiene 9 bzw. die Gelenkschienenschenkel 10, 11 eignet sich insbesondere Metall oder Kunststoff, wobei sich eine Kohlefaser-verstärkte dünne Platte als besonders günstig erwiesen hat, da diese unter Hitzeeinwirkung leicht formbar ist und nach dem Abkühlen eine große Federkraft bei dünner Materialstärke aufweist. Die Steifigkeit der Biegeschiene 9 in Mediolateralrichtung ist zusätzlich durch eine unebene Querschnittsform der Schenkel 10, 11 erhöht. Somit kann mit einer erfindungsgemäßen Vorrichtung mit einer Biegeschiene 9 zum einen ein hoher Tragekomfort erreicht werden, weil die Biegeschiene 9 individuell an den Fuß des Patienten anpassbar sind, und zum anderen wird hierdurch eine erhöhte Federhärte in Mediolateralrichtung erreicht, sodass im Wesentlichen hieraus resultierend ein überraschenderweise hoher Behandlungserfolg realisiert werden kann.

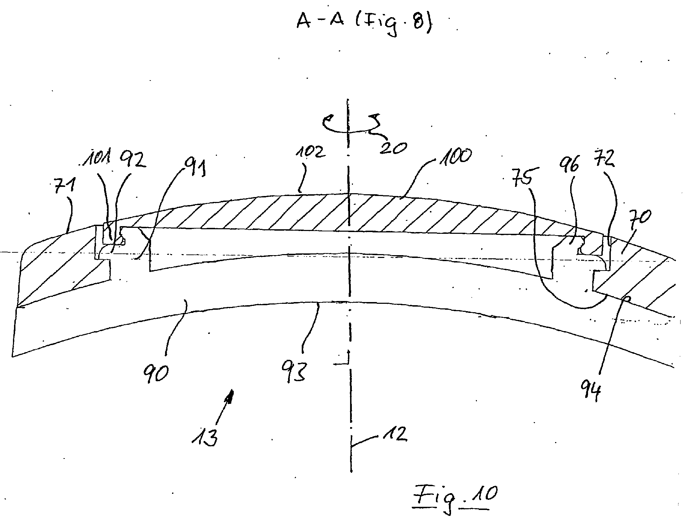

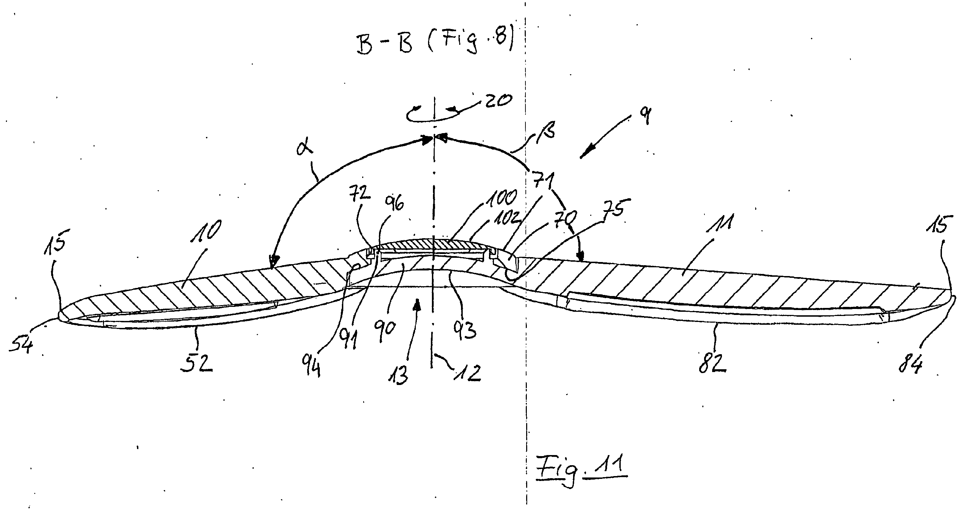

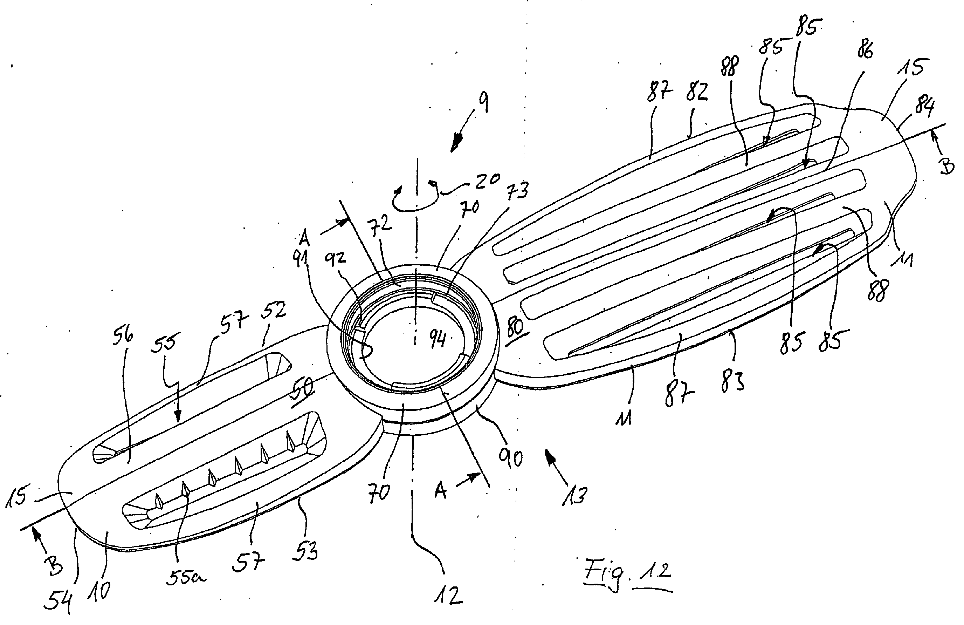

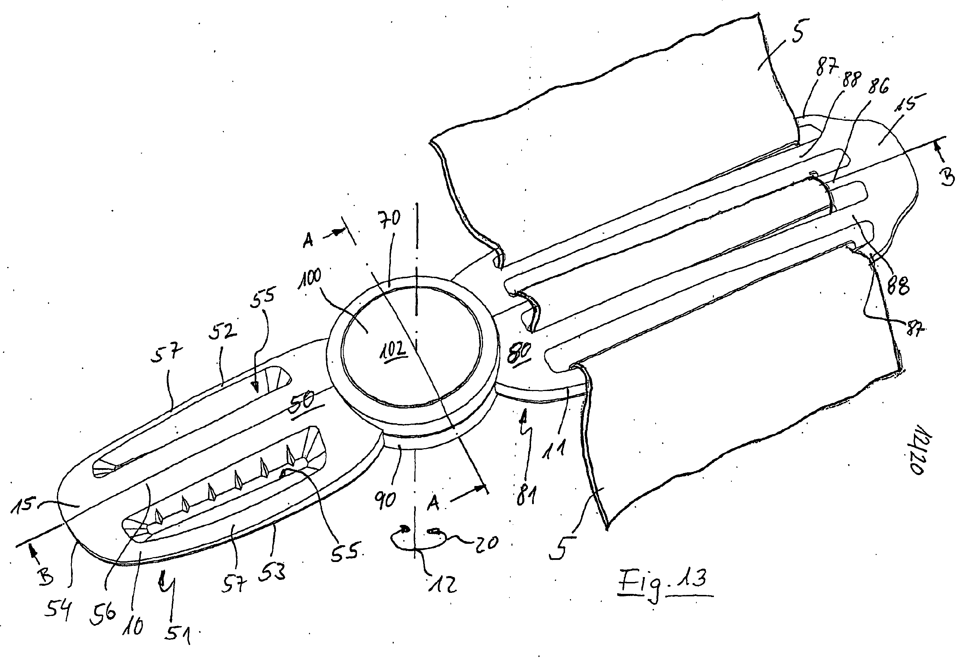

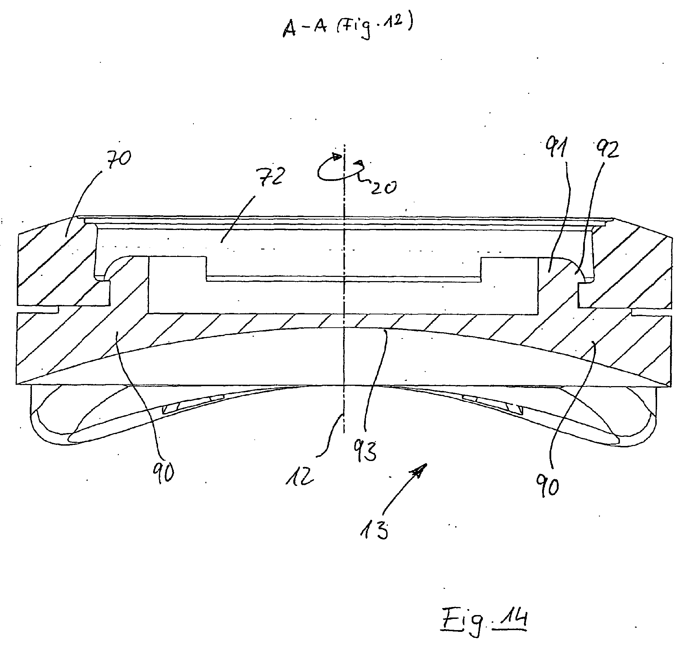

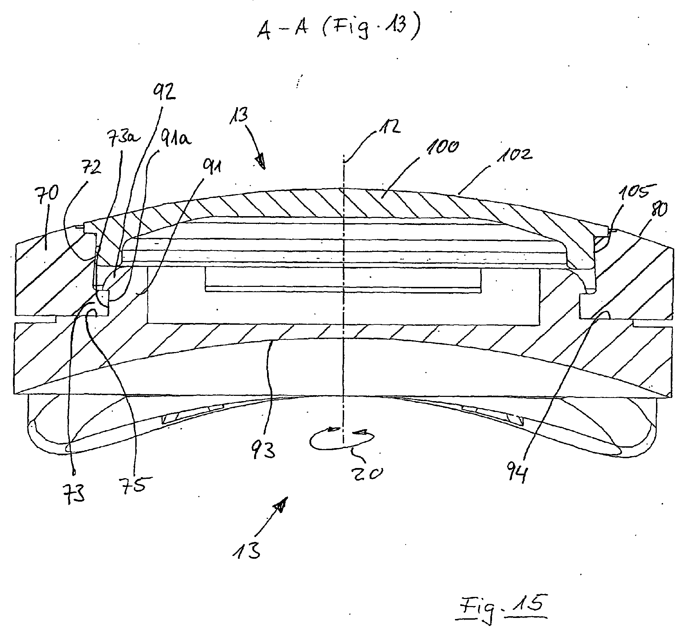

Weiterhin ist für eine erfindungsgemäße Vorrichtung 1 gewährleistet, dass an der Fußinnenseite bzw. an der zu korrigierenden Großzehe eine nur sehr dünne Materialauflage anzubringen ist, sodass die erfindungsgemäße Vorrichtung 1 ohne nennenswerte Beeinträchtigung in üblichem Schuhwerk getragen werden kann. Die Bewegungsfreiheit der Großzehe ist dadurch ebenfalls nicht eingeschränkt, da bei der erfindungsgemäßen Vorrichtung 1 die Möglichkeit besteht, die Zehe in der natürlichen Flexion-Extensionsrichtung 20 zu bewegen bzw. in diese Richtung ihre Bewegungsfreiheit nicht eingeschränkt ist (vgl. Fig. 5, 6). Somit eignet sich diese Vorrichtung 1 insbesondere für eine Dauerbehandlung sowohl tags als auch nachts, da der Patient keine nennenswerte Behinderung durch die Vorrichtung 1 verspürt.

Hinsichtlich ihrer Querschnittsraumform sind die Gelenkschienenschenkel 10, 11 uneben, d. h. beispielsweise gebogen mit konstanter Dicke aus einem Plattenmaterial geformt, wobei die Formgebung an die individuelle Fußkontur des Patienten angepasst ist. Weiterhin ist es selbstverständlich möglich, die Gelenkschienenschenkel 10, 11 in ihrem Querschnitt linsenförmig, insbesondere derart linsenförmig auszubilden, dass die Materialstärke zu den Rändern der Gelenkschienenschenkel 10, 11 hin abnimmt, sodass die geometrische Anpassung der Biegeschiene 9 an den Patientenfuß individuell weiter verbessert ist.

Gemäß einer weiteren Ausführungsform (nicht gezeigt) ist die erfindungsgemäße orthopädische Vorrichtung 1 zur Korrektur von Fehlstellungen mehrerer benachbarter Zehen weitergebildet, indem z. B. von der Ringbandage 6 um den Großzeh Zugmittel in Mediolateralrichtung abgehen und auf ein oder mehrere benachbarte Zehen einwirken, welche beispielsweise ebenfalls von einer Zehenringbandage umgeben sind. Somit kann die Korrekturkraft der Biegeschiene 9 in Mediolateralrichtung in einfacher Art und Weise vom Großzeh auf benachbarte Zehen übertragen werden. Durch geeignete Wahl der Materialstärke für die Gelenkschienenschenkel 10, 11 sowie durch eine geeignete Wahl eines Umfangsbeschnitts dieser Gelenkschienenschenkel 10, 11 sowie durch eine einfache Anpassbarkeit der Federvorspannung der Biegeschiene 9 in Mediolateralrichtung kann in einfacher Art und Weise Einfluss auf die Federhärte und somit auf die Zehenstellungs-korrigierende Kraft F1 genommen werden.

Eine individuelle Anpassung der korrigierenden Kraft F1 an spezifische Bedürfnisse des Patienten ist somit mit einfachen, in jeder orthopädischen Werkstatt vorhandenen Mitteln, z. B. durch Anpassung der Umfangskontur der Schenkel in 10, 11 oder der Veränderung der Querschnittskontur der Schenkel 10, 11 und/oder der Gelenkeinrichtung 13 möglich.

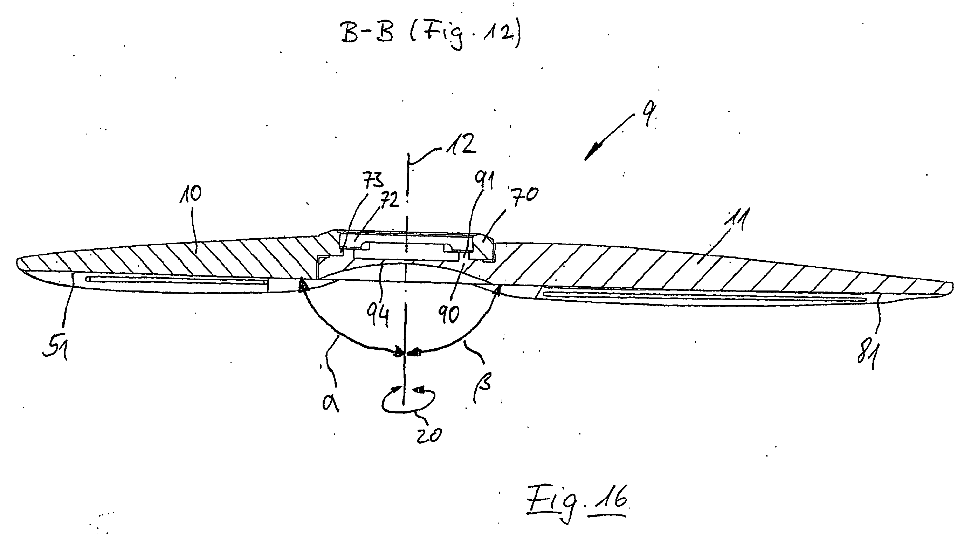

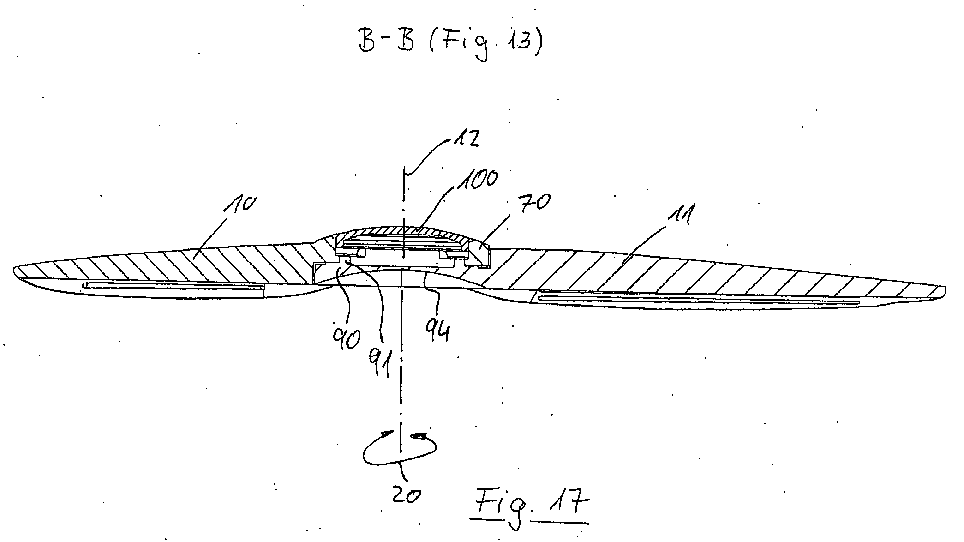

Eine weitere Ausführungsform der Gelenkschiene 9 der erfindungsgemäßen Vorrichtung 1 (Fig. 7) weist ebenfalls einen ersten Gelenkschienenschenkel 10 und einen zweiten Gelenkschienenschenkel 11 auf, welche mittels einer Gelenkeinrichtung 13 um eine Schwenkachse 12 schwenkbar gelenkig miteinander verbunden sind. Der erste Gelenkschienenschenkel 10 ist im Falle der Anordnung der Vorrichtung 1 im Bereich des Großzehs des Patienten an der Fußinnenseite angeordnet. Der zweite Gelenkschienenschenkel 11 ist in diesem Fall im Bereich des Mittelfußes des Patienten an der Fußinnenseite angeordnet. Der erste Gelenkschienenschenkel 10 weist eine vom Fuß wegweisende Außenseite 50 und eine zum Fuß hinweisende Innenseite 51 auf. Der erste Gelenkschienenschenkel 10 weist eine in Längs- und Querrichtung gewölbte, im Wesentlichen plattenförmige Raumform mit einer ersten Längsbegrenzungskante 52 und einer zweiten Längsbegrenzungskante 53 sowie einer Schmalbegrenzungskante 54 auf. Benachbart zu den Längsbegrenzungskanten 52, 53 verlaufend besitzt der Gelenkschienenschenkel 10 schlitzförmige Öffnungen 55, sodass ein Mittelsteg 56 und Randstege 57 gebildet sind.

Die Wölbungen des Gelenkschienenschenkels 10 in seiner Längs- und Querrichtung sind an die anatomischen Gegebenheiten eines Fußes angepasst.

Zur Befestigung des ersten Gelenkschienenschenkels 10 an der Großzehe des Patienten ist die Ringbandage 6 vorgesehen, welche mit dem Gelenkschienenschenkel 10 verbunden ist. Die Ringbandage 6 besitzt einen Schlaufenkörper 60 und freie Enden 61. Der Schlaufenkörper 60 dient zur Umschlingung der Großzehe des Patienten. Die freien Enden 61 sind von der Innenseite 51 her durch die schlitzförmigen Öffnungen 55, diese durchgreifend, geführt, und die Randstege 57 umgreifend mit einer Außenseite des Schlaufenkörpers 60 lösbar befestigt. Somit ist eine sichere Befestigung des Gelenkschienenschenkels 10 an der Großzehe des Patienten und eine Anpassbarkeit der Größe des Schlaufengröße 60 an unterschiedliche Größen der Großzehen gewährleistet.

Am gelenkseitigen Ende 16 des ersten Gelenkschienenschenkels 10 weist dieser einen Gelenkring 70 auf, dessen Mittelachse die Schwenkachse 12 ist. Der Gelenkring 70 ist bevorzugt einstückig mit dem ersten Gelenkschienenschenkel 10 verbunden. Der Gelenkring 70 besitzt eine Außenseite 71, eine Innenseite 72 sowie eine sich von der Innenseite 72 ein Stück in Richtung zur Schwenkachse 9 erstreckende Ringstufe 73 auf.

Der zweite Gelenkschienenschenkel 11 besitzt eine Außenseite 80 und einen Innenseite 81 sowie eine erste Längsbegrenzungskante 82 und eine zweite Längsbegrenzungskante 83 sowie eine Schmalseitenbegrenzungskante 84. Der zweite Gelenkschienenschenkel 11 ist hinsichtlich seiner Raumform im Wesentlichen als ein in Längsrichtung und Querrichtung gewölbter plattenförmiger Körper aufgebaut, wobei die Wölbungen in Längs- und Querrichtung an die anatomischen Gegebenheiten im Bereich des Mittelfußes an einer Fußinnenseite angepasst sind. Zum freien Ende 15 hin sowie zu den Längsbegrenzungskanten 82, 83 hin ist der zweite Gelenkschienenschenkel 11 wie auch der erste Gelenkschienenschenkel 10 sich jeweils in der Materialstärke verjüngend ausgebildet, sodass z. B. eine im Querschnitt in etwa linsenförmige Raumform ausgebildet ist. In etwa parallel zu den Längsbegrenzungskanten 82, 83 weist der zweite Gelenkschienenschenkel 11 schlitzförmige Öffnungen 85 auf, sodass ein Mittelsteg 86 sowie Randstege 87 und zwischen den Randstegen 87 und dem Mittelsteg 86 angeordnete Zwischenstege 88 ausgebildet sind.

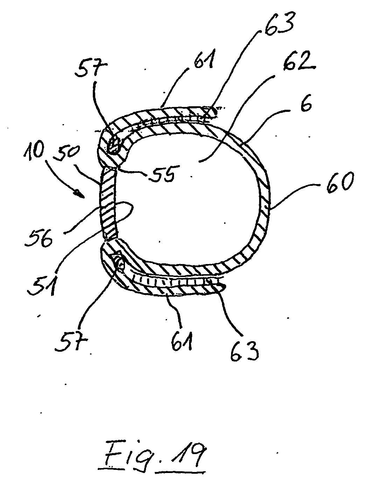

Das gelenkseitige Ende 16 des zweiten Gelenkschienenschenkels 11 ist bevorzugt mit dem Gelenkschienenschenkel 11 einstückig verbunden und ist als Gelenkteller 90 ausgebildet, welcher mit dem Gelenkring 70 des ersten Gelenkschienenschenkels 10 die Gelenkeinrichtung 13 bildet. Wenn die erfindungsgemäße Vorrichtung von einem Patienten getragen wird und an dessen Fuß befestigt ist, ist der Gelenkteller 90 der Fußhaut zugewandt und liegt auf dieser auf. Der Gelenkring 70 ist außenseitig gegen den Gelenkteller 90 gesetzt. Der Gelenkteller 90 weist konzentrisch zur Ringöffnung des Gelenkrings 7 einen Ringsteg 91 auf, welcher sich vom Gelenkteller 90 ein Stück in Richtung des Innenraums des Gelenkrings 70 erstreckt. Der Ringsteg 91 sitzt spielfrei oder nahezu spielfrei innerhalb der Ringstufe 73 des Gelenkrings 70, sodass eine radiale Führung des Gelenktellers 90 und des Gelenkrings 70 zueinander gewährleistet ist. Zur axialen Festlegung des Gelenkrings 70 bezüglich des Gelenktellers 90 sind an den Ringsteg 91 Rastnasensegmente 92 (Fig.9) angeformt, welche mit einer Oberseite der Ringstufe 73 zusammenwirken und somit eine axiale Festlegung der Gelenkschienenschenkel 10 und 11 zueinander gewährleisten. Die Anordnung der Rastnasensegmente 92 wird im Zusammenhang mit der Fig. 9 näher beschrieben werden. Um ein sicheres Fügen der Gelenkeinrichtung durch Aufstecken des Gelenkrings 70 auf den Gelenkteller 90 und Verrasten der Rastnasensegmente 92 mit der Ringstufe 73 zu gewährleisten, ist es zweckmäßig, die Rastnasensegmente 92 nicht vollumfänglich am Ringsteg 91 anzuordnen, damit durch die Materialelastizität der Gelenkring 70 beim Fügen auffedern kann.

Gemäß einer besonders bevorzugten Ausführungsform (Fig. 9) weist der Gelenkteller 90 eine im Querschnitt Kalottenförmige, insbesondere Kugelkalotten-förmige Raumform auf und besitzt eine zum Fuß hingewandte Seite 93 und eine zum Gelenkring 70 hingewandte Außenseite 94, welche korrespondierend zur Innenseite 93 ebenfalls, insbesondere im Bereich außerhalb des Ringstegs 91, Kalotten-förmig oder teilbereichsweise Kalotten-förmig ausgebildet ist. Korrespondierend hierzu weist der Gelenkring 70 einer der Außenseite 71 gegenüberliegende Innenseite 75 auf, welche in ihrer Raumform derart ausgebildet ist, dass sie flächig auf der korrespondierenden Innenseite 93 des Gelenktellers 90 im Bereich außerhalb des Ringstegs 91 aufliegt.

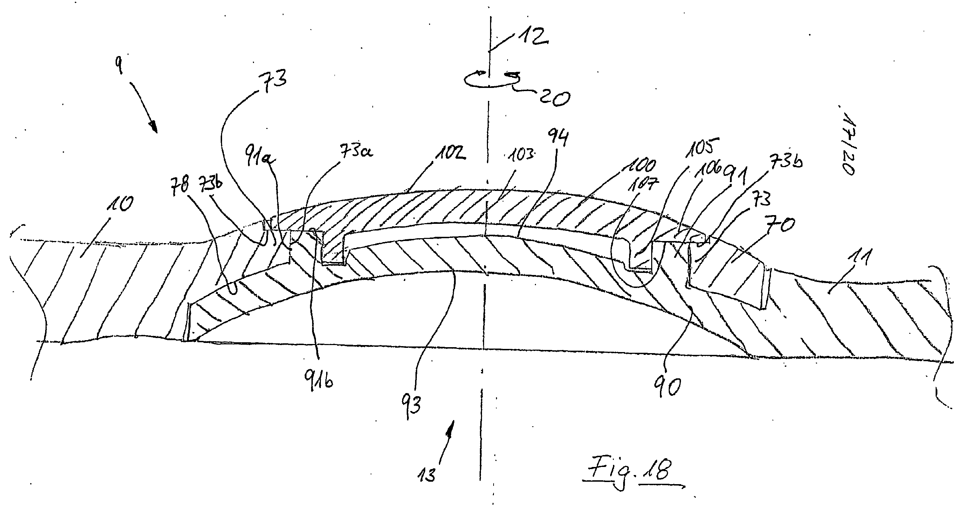

Zusätzlich zu den Rastnasensegmenten 92 weist der Ringsteg 91 an seinem freien Ende einen weiteren Rastring 96 oder Rastringsegmente auf, welche zur Befestigung einer deckelartigen Verschlusseinrichtung 100 (vergleiche Fig. 8, 10) dienen.

Die deckelartige Verschlusseinrichtung 100 weist zum Rastringsteg 96 korrespondierende Gegenrastmittel 101 auf, sodass die Verschlusseinrichtung formschlüssig mit dem Gelenkteller 90 bzw. dessen Ringsteg 91 verbindbar ist. Die Verschlusseinrichtung 100 weist eine Außenseite 102 auf, die in ihrer Raumform derart ausgebildet ist, dass ein harmonischer Übergang von der Oberseite 73 des Gelenkrings 70 gewährleistet ist. Die Verschlusseinrichtung 100 dient dazu, die offene Gelenkeinrichtung zu verschließen und somit vor Verschmutzung und/oder Beschädigung zu schützen. Bevorzugt ist die Verschlusseinrichtung 100 mit Spiel zur .Innenseite 72 des Gelenkrings eingesetzt, um unerwünschte Geräusche und/oder überhöhte Reibung zu vermeiden.

Somit ist in einfacher Art und Weise eine leicht zu montierende Gelenkeinrichtung 13 geschaffen, welche eine außerordentlich hohe Biege-Belastbarkeit bei gleichzeitig geringer Materialdicke aufweist und zudem mit nur wenigen Bauteilen auskommt. Außerdem bewirkt die Ausbildung der Gelenkeinrichtung 13 nach Art eines Ringgelenks, dass in die Gelenkeinrichtung 13 hohe Biegekräfte und Biegemomente einleitbar sind, ohne deren Leichtgängigkeit zu beeinträchtigen oder derer Verschleiß übermäßig zu fördern. Dies ist vor allem auf die große Stützweite, welche durch ein außenseitiges Eingreifen der Rastnasensegmente 92 in eine innenseitige Ringstufe 72 des Gelenkrings 70 ermöglicht wird, zurückzuführen.

Somit ist die Gelenkschiene 9 im Wesentlichen aus lediglich zwei Einzelteilen gebildet, welche die Funktion der Schiene 9 sicherstellen. Die Verschlusseinrichtung 100 besitzt lediglich eine Verschlussfunktion und ist zur Ausbildung des Gelenks nicht notwendig. Besonders vorteilhaft bei einer solchen Ausgestaltung der Gelenkeinrichtung 13 ist, dass die im Bereich des Großzehengrundgelenks an einer eventuell vorhandenen Pseudoexostose des Patientenfußes anliegende Innenseite 93 des Gelenktellers 90 eine glatte Oberfläche aufweist, d.h. keine überstehende oder vorstehende Teilbereiche oder Kanten aufweist, was den Tragekomfort erheblich erhöht. Durch die Kalotten-förmige Ausgestaltung ist die größtmögliche Druckentlastung empfindlicher Bereiche des Fußes (Pseudoexostose) und somit eine Schmerzentlastung des Patienten sichergestellt, da die eingeleitete Druckkraft F₂ auf eine große Fläche verteilt wird. Entlang des Längsverlaufes (Fig. 11) der Gelenkschiene 9 ist dafür gesorgt, dass die Innenseite 51, die Innenseite 93 und die Innenseite 81 der Gelenkschienenbauteile 10, 11 einen stetigen, stufenfreien und geschmeidigen Verlauf aufweisen, sodass der Tragekomfort am Fuß weiter verbessert ist. Die schlitzförmigen Öffnungen 55 bzw. 85 weisen ggf. an deren Flankenflächen Verzahnungen 55a auf, welche eine Rutschsicherung für die durchzufädelnden Ringbandagen 6 darstellen.

Die Gelenkschienenschenkel 10 und 11 schließen in ihrer Längserstreckung mit der Schwenkachse 12 einen Winkel α bzw. β ein (vergleiche Fig. 11). Die Winkel α und β sind derart gewählt, dass die Gelenkschiene 9 derart an einen Patientenfuß anlegbar ist, dass die Schwenkachse 12 in etwa mit der anatomischen Gelenkachse des Großzehengrundgelenks fluchtet, sodass bei einer Schwenkbewegung des Gelenkes mit am Fuß befestigten Gelenkschienenschenkeln 10 und 11 eine kinematisch weitgehend an die anatomischen Bedingungen des Großzehengrundgelenks angepasste Schwenkbewegung der Gelenkschiene 9 ermöglicht wird. Weiterhin sind die Winkel α und β derart gewählt, dass für eine zur Fußaußenseite hin schief gestellte Großzehe (Hallux-valgus-Stellung) eine Korrektur in Richtung hin zu einer orthopädischen Normalstellung erreichbar ist. Selbstverständlich liegt es auch im Bereich der Erfindung, die Winkel α und β derart zu wählen, dass eine seltener auftretende so genannte Hallux-varus-Stellung, d. h. eine Zehenschiefstellung, die ausgehend von der Normalstellung hin zur Fußinnenseite verschoben ist, korrigiert werden kann. Die Auswahl der Winkel α und β nach den oben genannten Kriterien ist selbstverständlich für alle in dieser Anmeldung skizzierten Ausführungsformen möglich und nicht auf die Ausführungsform gemäß den Fig. 7 bis 11 beschränkt. Je nach Grad der auftretenden Zehenschiefstellung und der erwünschten Korrekturkraft hat sich für den Winkel α ein Bereich zwischen 75° und 115° bewährt. Innerhalb dieses Bereichs ist ein Großteil der auftretenden Hallux-valgus- und Hallux-varus-Schiefstellungen korrigierbar. Selbstverständlich kann in extremen Einzelfällen auch ein Winkel α außerhalb dieses Bereichs notwendig werden. Für den Winkel β hat sich ein Winkel von etwa 70° bis 110° bewährt, um einen Großteil der üblicherweise auftretenden Hallux valgus und/oder Hallux-varus-Stellungen zu korrigieren. Selbstverständlich gilt auch für den Winkel β, dass er in Einzelfällen größer oder kleiner zu wählen ist.

Eine weitere Ausführungsform der Gelenkschiene 9 der erfindungsgemäßen Vorrichtung 1 (Fig. 12) ist im Wesentlichen identisch aufgebaut wie die Ausführungsform gemäß den Fig. 7 und beruht hinsichtlich der Korrektur der Zehenfehlstellungen auf dem gleichen Wirkprinzip. Im Unterschied zur Ausführungsform gemäß den Fig. 7 bis 11 ist bei der Ausführungsform gemäß den Fig. 12 bis 17 lediglich die Gelenkeinrichtung 13 etwas anders gestaltet. Die Innenseite 72 des Gelenkrings 70 ist in ihrer axialen Längserstreckung etwas größer ausgebildet. Zudem verfügt der Ringsteg 91 des Gelenktellers 90 lediglich über die Rastnasensegmente 92, nicht jedoch über die aufgesetzten Rastnasensegmente 96 zur Halterung der Verschlusseinrichtung 100.

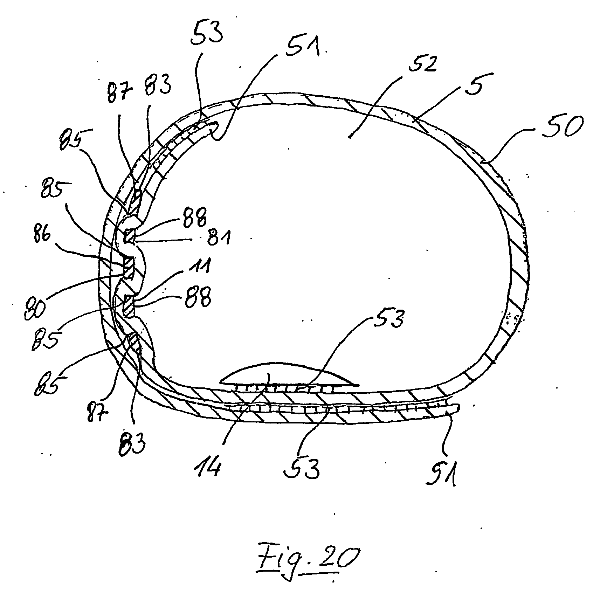

Die Verschlusseinrichtung 100 (vergleiche Fig. 13) ist derart in den Gelenkring 70 eingesetzt, dass eine Ringaußenwandung 105 der Verschlussvorrichtung 100 mit der Innenseite 72 des Gelenkrings 70 derart zusammenwirkt, dass die Verschlussvorrichtung 100 mittels eines Klemmsitzes, ggf. auch mittels eines Klebsitzes bezüglich des Gelenkrings 70 befestigt ist. Bei dieser Konstruktion ist von Vorteil, dass die Verschlusseinrichtung 100 nicht relativ zum Gelenkring 70 bewegt wird, sodass eine Scheuerwirkung auf einen ggf. die Gelenkschiene mitsamt der Gelenkeinrichtung 16 umgebenden Strumpf oder eine Innenseite des Schuhs minimiert ist. Somit ist sichergestellt, dass zwischen den Teilbereichen 50, 102, 80 der Außenseite der Gelenkschiene 9 bei Betätigung der Gelenkschiene keine Relativbewegung der Flächenabschnitte 80, 100, 70 zueinander im Bereich der Gelenkeinrichtung 13 stattfindet. Ein weiterer Unterschied ist, dass die Gelenkgleitflächen 94 des Gelenktellers 90 und die korrespondierende Fläche 75 des Gelenkrings 70 als planare Ringflächen ausgebildet sind, was eine Konstruktionsvereinfachung dahingehend darstellt, dass die radiale Führung des Gelenkrings 70 bezüglich des Gelenktellers 90 ausschließlich über eine Außenseite 91a des Ringstegs 91 und über eine Innenseite 73a der Ringstufe 73 erfolgt.

Selbstverständlich liegt es auch im Bereich der Erfindung, die Verschlusseinrichtung 100 nach Art der Ausführungsform gemäß den Fig. 12 bis 17 auf eine Ausführungsform der Gelenkeinrichtung 13 gemäß den Fig. 7 bis 12 zu übertragen. Gleiches gilt für die Ausbildung der Gleitflächen 75, 94 hinsichtlich der Übertragung des Prinzips gemäß den Fig. 7 bis 12 auf eine Gelenkeinrichtung 13 gemäß den Fig. 13 bis 17.

Aus einem Längsschnitt der Ausführungsform gemäß den Fig. 12 und 13 (Fig. 16) geht hervor, dass der wirksame Korrekturwinkel β zwischen der Schwenkachse 12 und den am Fuß anliegenden wirksamen Bereichen der Gelenkschiene 9 jeweils 90° ist. Weiterhin weisen die Gelenkschienenschenkel 10 und 11 in ihrem wirksamen Längsverlauf entlang ihrer Seiten 51 und 81 eine gerade Raumform auf, welche nicht in besonderem Maße an den anatomischen Verlauf eines Fußes bzw. einer Großzehe angepasst ist. Somit stellt eine derartige Ausführungsform der Gelenkschiene eine vereinfachte, aber für eine Großzahl der zu behandelnden Fälle wirkungsvolle Ausgestaltung der Gelenkschiene 9 dar. Der korrespondierende Winkel α und β in Fig. 11 wurde der Einfachheit halber zwischen der Schwenkachse 12 und der Außenlinie eingezeichnet, was jedoch keinen wesentlichen Unterschied macht. Gemeint ist in jedem Fall der wirksame Winkel einer gedachten Gelenkschienenschenkel-Wirkachse zur Schwenkachse 12, wobei im Falle einer vollständig frei geformten, an die anatomischen Gegebenheiten des menschlichen Fußes angepassten Gelenkschiene gemäß Fig. 11 die zeichnerische Festlegung einer solchen Wirksachse nur eine grobe Schätzung sein kann. Somit wird festgestellt, dass die Winkel α und β gemäß Fig. 16 und die Winkel α und β gemäß Fig. 11 im Prinzip die gleichen Wirkwinkel darstellen sollen.

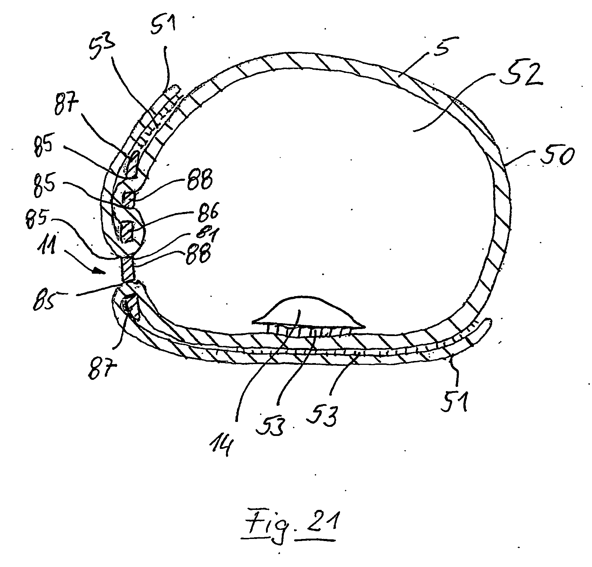

In der Längsschnittdarstellung gemäß Fig. 17 ist die Ausführungsform der Gelenkschiene 9 gemäß den Fig. 12 und 13 mit einer eingesetzten Verschlusseinrichtung 100 gezeigt.

Eine weitere Ausführungsform der Gelenkeinrichtung 13 (Fig. 18) der Gelenkschiene 9 der erfindungsgemäßen Vorrichtung 1 ist ebenfalls als so genanntes Ringscheibengelenk mit einem Gelenkteller 90 und einem Gelenkring 70 aufgebaut. Der Gelenkteller 90 weist eine zur Fußhaut weisende Innenseite 93 und eine gegenüberliegende Außenseite 94 auf. Von der Außenseite 94 erstreckt sich ein Stück in Richtung weg vom Fuß des Patienten ein Ringsteg 91, welcher eine Ringstegaußenfläche 91a besitzt. Die Ringstegaußenfläche 91a dient zur radialen Führung des Gelenkrings 70 und wirkt spielfrei oder mit geringem Spiel mit einer Gelenkringinnenseite 73a zusammen.

Der Gelenkteller 90 ist wie in den vorangegangenen Ausführungsformen bevorzugt Kalotten-förmig, insbesondere Kugelkalotten-förmig ausgebildet und weist außerhalb des Ringstegs 91 einen Teilbereich der Außenseite 94 auf, welcher als Gelenkgleitfläche dient und mit einer korrespondierenden Gelenkgleitfläche 78 des Gelenkrings 70 zusammenwirkt. Die Gelenkgleitflächen haben eine Kalottenabschnitts-förmige Raumform. Bei der Ausführungsform gemäß der Fig. 18 ist die Höhe der Ringstufe 73 des Gelenkrings 70 derart gewählt, dass eine Oberseite 73b der Ringstufe mit einer Oberseite 91b des Ringstegs 91 bündig abschließt oder etwas übersteht. Somit ist durch das Zusammenwirken der Ringstege 91 und des Gelenkrings 70 mit deren Flächen 91a und 73a eine radiale Führung der Gelenkschienenschenkel 10, 11 gewährleistet. Die axiale Führung der Gelenkschienenschenkel 10, 11 zueinander wird über die Gleitfläche 78 und deren korrespondierenden Gleitflächenabschnitt der Außenseite 94 des Gelenktellers 90 in einer Richtung sichergestellt. In der zu dieser Richtung entgegengesetzten Richtung ist die axiale Festlegung der Gelenkschienenschenkel 10, 11 bzw. des Gelenkrings relativ zum Gelenkteller 90 wie folgt sichergestellt.

Die Verschlusseinrichtung 100 ist zusätzlich zu ihrer Verschlussfunktion der Gelenkeinrichtung 13 als Axiallager weitergebildet. Die Verschlusseinrichtung 100 weist einen Verschlusseinrichtungskörper 103 auf, welcher wie in den vorbeschriebenen Ausführungsformen im Wesentlichen die Gelenkeinrichtung 13 bedeckt und somit vor Verschmutzungen schützt. Vom Verschlusseinrichtungskörper 103 erstreckt sich gegenüberliegend von der Oberseite 102 ein Ringsteg 105, welcher im Durchmesser derart bemessen ist, dass er innerhalb des Ringstegs 91 des Gelenktellers 90 in den vom Ringsteg 91 umgrenzten Raum einsteckbar ist. Außerhalb des Ringstegs 105 weist die Verschlusseinrichtung 100 einen umlaufenden Vorsprung 106 auf, welcher in radialer Richtung derart bemessen ist, dass er den Ringsteg 91 radial ein Stück überragt und auf der Ringstufenoberseite 73b der Ringstufe spielfrei oder mit geringem axialen Spiel aufliegt. Somit dient eine Unterseite 107 des Ringvorsprungs 106, welche mit der Oberseite 73b der Ringstufe 73 zusammenwirkt, als axiales Widerlager. Damit durch eine solche Gelenkkonstruktion hohe Biegekräfte, welche die Gelenkschiene 9 ausüben muss, beschädigungsfrei und dauerhaft übertragen werden können, ist die Verschlussvorrichtung 100 zweckmäßigerweise mit dem Gelenkteller bzw. dessen Ringsteg 91 an einer oder mehreren deren Berührungsflächen verklebt oder anderweitig sicher befestigt.

Auch diese Gelenkeinrichtung 9 hat den besonderen Vorteil, dass die an den besonders empfindlichen Bereichen des Fußes anliegenden Flächen 93, z. B. im Bereich einer Pseudoexostose, glatt ausgebildet sind, d. h. dass keine Überstände oder Vertiefungen die dortigen Hautbereiche reizen. Zudem weist die Gelenkausführung gemäß Fig. 18 eine besonders hohe Biegesteifigkeit auf und ist auch durch missbräuchliches Überbiegen der Schiene nicht ohne weiteres zerlegbar, d. h. dass durch unsachgemäße Behandlung durch den Patienten, z. B. durch Tordieren, Überbiegen oder dergleichen es ausgeschlossen ist, dass die beiden Gelenkschienenschenkel 10, 11 voneinander trennbar sind.

Als bevorzugter Werkstoff für die Gelenkschienen hat sich neben Kunststoff, insbesondere ein schlagzäher und hautverträglicher Kunststoff, z. B. aus der Gruppe der Polykarbonate, bewährt.

Im Folgenden wird anhand der Fig. 19 bis 21 die Befestigung der Gelenkschiene 9 an dem Patientenfuß und die Führung bzw.

Fädelung der Ringbandagen 5, 6 durch die Schlitzöffnungen 55, 85 der Gelenkschienen 9 näher beschrieben.

Die Ringbandage 6 im Bereich des Großzehs (Fig. 19)dient als Befestigungseinrichtung und weist den Schlaufenkörper 60 sowie freie Enden 61 auf. Der Schlaufenkörper 60 umgrenzt zusammen mit dem ersten Gelenkschienenschenkel 10 einen Innenraum 62, in dem die Zehe des Patienten angeordnet ist. Die freien Enden 61 sind von der Innenseite 51 her durch die schlitzförmigen Öffnungen 55 geführt und um den Randsteg 57, diesen umschließend, herumgeschlagen. Somit kommt das freie Ende 61 der Ringbandage 6 benachbart zum Gelenkschienenschenkel 10 außenseitig mit dem Schlaufenkörper 60 zur Anlage. Das freie Ende 61 ist zweckmäßigerweise mittels eines Klettverschlusses 63 an der Außenseite des Schlaufenkörpers lösbar befestigt. Diese Art der Befestigung des freien Endes 61 kann auf beide freien Enden 61 der Ringbandage 6 angewendet werden. Es ist jedoch ggf. auch zweckmäßig, ein freies Ende 61 um einen Randsteg 57 herumzuschlagen und ggf. mit dem Schlaufenkörper 60 fest zu vernähen und nur ein weiteres freies Ende 61 mittels einer Klettverbindung oder dergleichen vergleichbare lösbare Flächenverbindung mit dem Schlaufenkörper 60 zu verbinden.

Die Ringbandage 5 dient als Befestigungseinrichtung und als Bandage zur Stützung und Wiederaufrichtung des Quergewölbes des Fußes und ist ebenfalls einen Schlaufenkörper 50 bildend mit dem zweiten Gelenkschienenschenkel 11 verbunden. Die Ringbandage 5 weist ebenfalls freie Enden 51 auf. Ein freies Ende 51 ist von der Innenseite 81 her durch die erste schlitzförmige Öffnung 85 nahe der Fußunterseite geführt und durchdringt diese, liegt außenseitig auf dem ersten Zwischensteg 88 auf und durchdringt die zweite Schlitzöffnung 85 von der Außenseite 80 her, liegt im Bereich des Mittelsteges 86 auf der Innenseite 81 auf und durchdringt die nächste schlitzförmige Öffnung 85 von der Innenseite 81 her hin zur Außenseite, liegt auf dem zweiten Zwischensteg 88 außenseitig an und durchdringt die letzte Schlitzöffnung 85 von der Außenseite 80 her nach innen. Das überstehende freie Ende 51 ist z.B. im Bereich des Ristes des Fußes anliegend angeordnet. Der restliche Bereich der Ringbandage 5, welcher nicht an der Fädelung durch den zweiten Gelenkschienenschenkel 11 beteiligt ist, ist den Fuß den Patienten umgebend um die Fußunterseite und die Fußoberseite geführt, überlappt den zweiten Gelenkschienenschenkel 11 im Bereich der Schlitzöffnungen 85 und ist im dem zweiten freien Ende 51 außenseitig am Schlaufenkörper 50 befestigt. Als Befestigungsmittel haben sich Klettverschlüsse, insbesondere Hakenklettverschlüsse oder Pilzklettverschlüsse 53 bewährt. Bei dieser Art der Fädelung der Ringbandage 5 durch den zweiten Gelenkschienenschenkel 11 und das anschließende Umgeben des gesamten Mittelfußes ist von besonderem Vorteil, dass die Randbegrenzungskanten 83 des Gelenkschienenschenkels 11 nicht direkt auf der Fußhaut aufliegen und somit eine Druckstellenbildung verhindert ist.

Gegebenenfalls kann es auch zweckmäßig sein, die zwei zentraler angeordneten Schlitzöffnungen 85 entfallen zu lassen und die Ringbandage 5 lediglich durch die äußersten Schlitzöffnungen 85 ohne den Umweg um den Mittelsteg 86 zu führen. Diese Ausführungsform hat den Vorteil, dass im Bereich des Gelenkschienenschenkels 11, in dem dieser am Fuß anliegt, weniger Unebenheiten und somit weniger Druckstellen am Fuß auftreten. Im Bereich des von Schlaufenkörper 50 umgebenen Innenraums 52, in dem der Mittelfußbereich des Patienten angeordnet ist, ist zweckmäßigerweise im Bereich der Fußunterseite des Patienten die Pelotte 14 mit der Ringbandage 5 ebenfalls über Klettverbindungselemente 53 verbunden.

Ein weiterer Vorteil dieser Art der Fädelung der Ringbandage 5 ist, dass zum Anlegen der Schiene 9 keine Fädelung durch die Öffnungen 85 des Gelenkschienenschenkels 11 notwendig ist, sondern lediglich die fertig gefädelte Ringbandage um den Fuß geschlagen werden muss und an dessen Unterseite festgezurrt und dort befestigt werden muss. Somit ist ein leichtes Einstellen oder Nachstellen der Spannung der Ringbandage 5 und somit deren Korrekturwirkung auf das Quergewölbe des Patientenfußes im Bereich des Mittelfußes ermöglicht.

Eine weitere Ausführungsform der Fädelung der Ringbandage 5 durch den zweiten Gelenkschienenschenkel 11 (gemäß Fig. 5) erfolgt derart, dass ein erstes freies Ende eine Schlitzöffnung 85 von der Innenseite 81 her durchgreifend, den Randsteg 87 im Bereich der Fußunterseite umgreifend geführt ist und außenseitig am Schlaufenkörper im Bereich der Fußunterseite, mit diesem mittels Verbindungselementen 53 verbunden ist. Das andere freie Ende 51 ist im Bereich der Fußoberseite von der Innenseite 81 her durch eine randliche Schlitzöffnung 85 nach außen geführt, liegt außenseitig am ersten Zwischensteg 88 an und durchdringt eine weitere schlitzförmige Öffnung 85 in Richtung zur Innenseite 81 hin, liegt innenseitig am Mittelsteg 86 an, umschlingt diesen durch eine weitere Öffnung 85 und ist außenseitig in Richtung zur Fußoberseite hin, den Gelenkschienenschenkel 11 teilweise überlappend, geführt. Das freie Ende 51 kann somit mittels Verschlusselementen 53 außenseitig am Schlaufenkörper 50 befestigt werden.

Die Ringbandagenführung gemäß Fig. 21 hat den Vorteil, dass die freien Enden in jedem Fall außenseitig am Schlaufenkörper 50 angeordnet sind, sodass zum Fuß hin keine Stufe in Höhe der Materialstärke zuzüglich der Verbindungselemente 51 entsteht und somit Druckstellen am Fuß vermieden werden. Auch bei dieser Ausführungsform ist es selbstverständlich möglich, das fußoberseitige freie Ende lediglich durch eine randliche Schlitzöffnung 85 zu führen, sodass dieses den Randsteg 87 umgreift und außenseitig auf dem Schlaufenkörper 50 befestigt werden kann. In diesem Fall entspricht die Fädelung bzw. Umschlingung der Ringbandage 5 der der Ringbandage 6 im Bereich des Großzehs. Auch kann bei dieser Ausführungsform im Bereich der Fußunterseite die Pelotte 14 mittels Klettelementen 53 an einer Innenseite des Schlaufenkörpers 50, insbesondere lösbar, befestigt werden.

Die nicht starre, in Grenzen schwimmende Anbindung der Vorrichtung 1 an die Innenseite des Fußes hat den Vorteil, dass sich die Vorrichtung 1 individuell an die anatomischen Randbedingungen anpassen kann, d.h. z.B. dass sich die Schwenkachse 12 der Gelenkschiene 9 nach dem Anlegen der Vorrichtung 1 selbsttätig fluchtend mit der anatomischen Gelenkachse des Großzehengrundgelenkes ausrichtet.

## Patentansprüche

1. Orthopädische Vorrichtung zur Korrektur von Zehenfehlstellungen, aufweisend eine erste Befestigungseinrichtung (8a, 6) im Bereich der Großzehe, eine zweite Befestigungseinrichtung (8b, 5) im Bereich des Mittelfußes und eine Biegeschiene (9), welche von den Befestigungseinrichtungen (8a, 8b, 5, 11) gehalten ist und sich entlang der Fußinnenseite erstreckt, **dadurch gekennzeichnet, dass** die Biegeschiene (9) als Gelenkbiegeschiene ausgebildet ist, welche in der Flexion-Extensionsrichtung (20) des oder der zu korrigierenden Zehen gelenkig ausgebildet ist und eine Gelenkeinrichtung (13) aufweist, welche eine Schwenkachse (12) aufweist, die in etwa der Gelenkachse des Großzehengrundgelenks in der Flexion-Extensionsrichtung entspricht.

2. Vorrichtung nach Anspruch 1, **dadurch gekennzeichnet, dass** die orthopädische Vorrichtung (1) einen Strumpf (2) oder dergleichen Umhüllungselement für einen Fuß aufweist, welcher die Befestigungseinrichtungen (8a, 8b, 5, 6) sowie die Biegeschiene (9) trägt.

3. Vorrichtung nach Anspruch 1 und/oder 2,**dadurch gekennzeichnet, dass** die Biegeschiene (9) Gelenkschienenschenkel (10, 11) aufweist, wobei die Gelenkschienenschenkel (10, 11) im Querschnitt linsenförmig ausgebildet sind.

4. Vorrichtung nach einem oder mehreren der vorangegangenen Ansprüche, **dadurch gekennzeichnet, dass** der Strumpf (2) im Zehenbereich offen ist.

5. Vorrichtung nach einem oder mehreren der vorangegangenen Ansprüche, **dadurch gekennzeichnet, dass** die Vorrichtung (1) eine Großzehenaufnahme, z. B. ein Großzehenfach (4) aufweist, welches mit dem Strumpf (2) einstückig verbunden oder an diesem Strumpf (2) angesetzt ist, wobei das Großzehenfach (4) den Großzeh umfänglich vollständig umgibt und im Bereich des freien Zehenendes offen oder geschlossen ausgebildet ist.

6. Vorrichtung nach einem oder mehreren der vorangegangenen Ansprüche, **dadurch gekennzeichnet, dass** die Vorrichtung (1) im Bereich des Mittelfußes eine erste Ringbandage (5) aufweist, welche den Mittelfuß vorzugsweise vollständig umgibt und mit dem Strumpf (2) in Verbindung steht.

7. Vorrichtung nach einem oder mehreren der vorangegangenen Ansprüche, **dadurch gekennzeichnet, dass** im Bereich des freien Endes der Großzehe eine zweite Ringbandage (6) das Großzehenfach (4) umgebend angeordnet ist, welche den Großzeh umfänglich vollständig umgibt.

8. Vorrichtung nach einem oder mehreren der vorangegangenen Ansprüche, **dadurch gekennzeichnet, dass** die Ringbandagen (5, 6) aus einem biegsamen, schmiegsamen, zirkulär zugstarren Material ausgebildet sind.

9. Vorrichtung nach einem oder mehreren der vorangegangenen Ansprüche, **dadurch gekennzeichnet, dass** im Bereich einer Fußinnenseite (7) sowohl die erste Ringbandage (5) als auch die zweite Ringbandage (6) teilbereichsweise nicht mit dem Strumpf (2) bzw. dem Großzehenfach (4) verbunden sind, sodass zwischen den Ringbandagen (5, 6) und dem Großzehenfach (4) bzw. dem Strumpf (2) Befestigungs-/Aufnahmeeinrichtungen (8a, 8b) ausgebildet sind.

10. Vorrichtung nach einem oder mehreren der vorangegangenen Ansprüche, **dadurch gekennzeichnet, dass** die Befestigungseinrichtungen (8a, 8b) Einstecktaschen sind.

11. Vorrichtung nach einem oder mehreren der vorangegangenen Ansprüche, **dadurch gekennzeichnet, dass** die Biegeschiene (9) einen ersten Gelenkschienenschenkel (10) und einen zweiten Gelenkschienenschenkel (11) aufweist, welche um die Schwenkachse (12) schwenkbar mittels einer Gelenkeinrichtung (13) gelenkig verbunden sind, wobei sich von der Gelenkeinrichtung (13) aus der erste Gelenkschienenschenkel (10) bis in die Befestigungseinrichtung (8a) und der zweite Gelenkschienenschenkel (11) bis in die zweite Befestigungseinrichtung (8b) erstreckt.

12. Vorrichtung nach einem oder mehreren der vorangegangenen Ansprüche, **dadurch gekennzeichnet, dass** im Bereich der Fußsohle hinter den Grundgelenken der Zehen zur retrokapitalen Abstützung des Mittelfußes eine Spreizfußpelotte (14) eingesetzt ist.

13. Vorrichtung nach einem oder mehreren der vorangegangenen Ansprüche, **dadurch gekennzeichnet, dass** die Befestigungseinrichtungen (8a, 8b) als auf den Strumpf (2) aufgenähte oder anderweitig befestigte Taschen ausgebildet sind.

14. Vorrichtung nach einem oder mehreren der vorangegangenen Ansprüche, **dadurch gekennzeichnet, dass** der Strumpf (2) als Kompressionsstrumpf ausgebildet ist.

15. Vorrichtung nach einem oder mehreren der vorangegangenen Ansprüche, **dadurch gekennzeichnet, dass** die Gelenkschienenschenkel (10, 11) jeweils ein freies Ende (15) und ein gelenkseitiges Ende (16) aufweisen, wobei die gelenkseitigen Enden (16) in etwa eine Kugelkalottenraumform aufweisen und zueinander derart korrespondierend ausgebildet sind, dass die jeweils gelenkseitigen Enden (16) der Gelenkschienenschenkel (10, 11) formschlüssig passend ineinander legbar sind.

16. Vorrichtung nach einem oder mehreren der vorangegangenen Ansprüche, **dadurch gekennzeichnet, dass** die Gelenkschienenschenkel (10, 11) und die Gelenkeinrichtung (13) in ihrer Raumform an die Fußkontur des Patienten angepasst sind.

17. Vorrichtung nach einem oder mehreren der vorangegangenen Ansprüche, **dadurch gekennzeichnet, dass** die gelenkseitigen Enden (16) der Gelenkschienenschenkel (10, 11) eine um die Achse (12) rotationssymmetrische, zueinander korrespondierende Raumform aufweisen.

18. Vorrichtung nach einem oder mehreren der vorangegangenen Ansprüche, **dadurch gekennzeichnet, dass** die Gelenkschienenschenkel (10, 11) aus Metall oder Kunststoff, insbesondere aus einer Kohlefaser-verstärkten dünnen Platte ausgebildet sind.

19. Vorrichtung nach einem oder mehreren der vorangegangenen Ansprüche, **dadurch gekennzeichnet, dass** durch die Biegeschiene (9) zur Lateralisierung des Großzehs eine Kraft F1 in Richtung zur Fußinnenseite hin auf den Großzeh ausübbar ist.

20. Vorrichtung nach einem oder mehreren der vorangegangenen Ansprüche, **dadurch gekennzeichnet, dass** Mittel vorhanden sind, um die Kraft F1 auch auf ein oder mehrere benachbarte Zehen einwirken lassen zu können, wobei die Mittel Zugelemente sind, welche Zehenaufnahmen für mehrere Zehen eines Fußes miteinander verbinden.

21. Vorrichtung nach einem oder mehreren der vorangegangenen Ansprüche, **dadurch gekennzeichnet, dass** die Gelenkschienenschenkel (10, 11) eine in Längs- und Querrichtung gewölbte, im Wesentlichen plattenförmige Raumform mit einer ersten Längsbegrenzungskante (52, 82) und einer zweiten Längsbegrenzungskante (53, 83) sowie einer Schmalbegrenzungskante (54, 84) aufweisen, wobei im Bereich der Längsbegrenzungskanten parallel zu deren Verlauf schlitzförmige Öffnungen (55, 85) vorgesehen sind, sodass ein Mittelsteg (56, 86), Randstege (57, 87) und Zwischenstege (88) gebildet sind.

22. Vorrichtung nach einem oder mehreren der vorangegangenen Ansprüche, **dadurch gekennzeichnet, dass** die Ringbandagen (5, 6) mit den Gelenkschienenschenkeln (10, 11) verbunden sind.

23. Vorrichtung nach einem oder mehreren der vorangegangenen Ansprüche, **dadurch gekennzeichnet, dass** die Ringbandagen (5, 6) Schlaufenkörper (60, 50) und freie Enden (51, 61) aufweisen.

24. Vorrichtung nach einem oder mehreren der vorangegangenen Ansprüche, **dadurch gekennzeichnet, dass** die Gelenkeinrichtung (13) als Ringgelenk mit einem Gelenkring (70) und einem Gelenkteller (90) ausgebildet ist.

25. Vorrichtung nach einem oder mehreren der vorangegangenen Ansprüche, **dadurch gekennzeichnet, dass** der Gelenkring (70) einstückig mit dem ersten Gelenkschienenschenkel (10) verbunden ist und/oder der Gelenkteller (90) einstückig mit dem zweiten Gelenkschienenschenkel (11) verbunden ist.

26. Vorrichtung nach einem oder mehreren der vorangegangenen Ansprüche, **dadurch gekennzeichnet, dass** die Gelenkschienenschenkel (10, 11) sich jeweils zu ihren Randbereichen hin in der Materialstärke verjüngend ausgebildet sind und die am Fuß des Patienten anliegenden Flächenbereiche der Gelenkschiene (9) glatt ausgebildet sind.

27. Vorrichtung nach einem oder mehreren der vorangegangenen Ansprüche, **dadurch gekennzeichnet, dass** am Gelenkteller (90) ein Ringsteg (91) angeformt ist, welcher mit dem Gelenkring (70) zusammenwirkt, sodass eine radiale Führung des Gelenktellers (90) und des Gelenkrings (70) zueinander gewährleistet ist.

28. Vorrichtung nach einem oder mehreren der vorangegangenen Ansprüche, **dadurch gekennzeichnet, dass** zur axialen Festlegung des Gelenkrings (70) bezüglich des Gelenktellers (90) Verrastungselemente (92) vorgesehen sind, welche mit einer Stufe, insbesondere einer Ringstufe (73) zusammenwirken.

29. Vorrichtung nach einem oder mehreren der vorangegangenen Ansprüche, **dadurch gekennzeichnet, dass** die Gelenkeinrichtung (13) mittels einer Verschlusseinrichtung (100) abgedeckt ist, wobei die Verschlusseinrichtung (100) mittels Rastmitteln (96, 101) mit der Gelenkschiene (9) verbunden ist.

30. Vorrichtung nach einem oder mehreren der vorangegangenen Ansprüche, **dadurch gekennzeichnet, dass** die Gelenkschienenschenkel (10, 11) in Ihrer Längserstreckung mit der Schwenkachse (12) einen Winkel α, β einschließen, wobei die Winkel α und β derart gewählt sind, dass die Gelenkschiene (9) an einem Patientenfuß anlegbar ist, derart dass die Schwenkachse (12) der Gelenkeinrichtung (13) in etwa mit der anatomischen Gelenkachse des Großzehengrundgelenks fluchtet.

31. Vorrichtung nach einem oder mehreren der vorangegangenen Ansprüche, **dadurch gekennzeichnet, dass** der Winkel α zwischen 75° und 115° beträgt.

32. Vorrichtung nach einem oder mehreren der vorangegangenen Ansprüche, **dadurch gekennzeichnet, dass** der Winkel β etwa 70° bis 110° beträgt.

33. Vorrichtung nach einem oder mehreren der vorangegangenen Ansprüche, **dadurch gekennzeichnet, dass** die Verschlusseinrichtung (100) als Axiallager ausgebildet ist.

34. Vorrichtung nach einem oder mehreren der vorangegangenen Ansprüche, **dadurch gekennzeichnet, dass** die Gelenkschiene (9) aus Kunststoff, insbesondere aus schlagzähem und hautverträglichem Kunststoff, z. B. aus der Gruppe der Polykarbonate, ausgebildet ist.

35. Vorrichtung nach einem oder mehreren der vorangegangenen Ansprüche, **dadurch gekennzeichnet, dass** die Bandagen (5, 6) zum Mittelfuß bzw. zum Großzeh des Patienten hin in ihrer Führung bzw. Fädelung durch die Gelenkschienenschenkel (10,11) stufenfrei angeordnet sind.

36. Vorrichtung nach einem oder mehreren der vorangegangenen Ansprüche, **dadurch gekennzeichnet, dass** die Vorrichtung (1) am Fuß nicht starr angebunden ist, sodass sich beim Tragen die Vorrichtung (1) individuell an die anatomischen Randbedingungen des Patientenfußes hinsichtlich der Lage der Gelenkachse (12) der Gelenkeinrichtung (13) anpassen kann.

## Claims

1. Orthopaedic device for correcting abnormal positions of the toes, comprising a first fixing device (8a, 6) in the region of the big toe, a second fixing device (8b, 5) in the region of the metatarsus, and a flexion splint (9) which is held by the fixing devices (8a, 8b, 5, 11) and extends along the inner side of the foot, **characterized in that** the flexion splint (9) is designed as an articulated flexion splint which is articulated in the flexion/extension direction (20) of the toe or toes to be corrected and has an articulation device (13) with a pivot axis (12) which corresponds approximately to the axis of articulation of the base joint of the big toe in the flexion/extension direction.

2. Device according to Claim 1, **characterized in that** the orthopaedic device (1) comprises a stocking (2) or other such sheath element for a foot, which stocking carries the fixing devices (8a, 8b, 5, 6) and also the flexion splint (9).

3. Device according to Claim 1 and/or 2, **characterized in that** the flexion splint (9) has articulated splint limbs (10, 11), wherein the articulated splint limbs (10, 11) are lenticular in cross section.

4. Device according to one or more of the preceding Claims, **characterized in that** the stocking (2) is open in the toe region.

5. Device according to one or more of the preceding Claims, **characterized in that** the device (1) comprises a big toe holder, for example a big toe section (4), which is connected in one piece with the stocking (2) or is attached to this stocking (2), wherein the big toe section (4) completely surrounds the circumference of the big toe and is designed to be open or closed in the region of the free end of the toe.

6. Device according to one or more of the preceding Claims, **characterized in that** the device (1) comprises in the region of the metatarsus a first annular bandage (5) which preferably completely surrounds the metatarsus and is joined to the stocking (2) .

7. Device according to one or more of the preceding Claims, **characterized in that** a second annular bandage (6) is arranged in the region of the free end of the big toe in a manner completely surrounding the big toe section (4).

8. Device according to one or more of the preceding Claims, **characterized in that** the annular bandages (5, 6) are made of a flexible, pliable material which is resistant to tensile loading in the circular direction.

9. Device according to one or more of the preceding Claims, **characterized in that**, in the region of a foot inner side (7), both the first annular bandage (5) and the second annular bandage (6) are not joined to the stocking (2) or respectively to the big toe section (4) in some areas, so that fixing/holding devices (8a, 8b) are formed between the annular bandages (5, 6) and the big toe section (4) or the stocking (2).

10. Device according to one or more of the preceding Claims, **characterized in that** the fixing devices (8a, 8b) are insertion pockets.

11. Device according to one or more of the preceding Claims, **characterized in that** the flexion splint (9) comprises a first articulated splint limb (10) and a second articulated splint limb (11) which are connected in an articulated manner by means of an articulation device (13) such that they can pivot about the pivot axis (12), wherein, starting from the articulation device (13), the first articulated splint limb (10) extends into the fixing device (8a) and the second articulated splint limb (11) extends into the second fixing device (8b).

12. Device according to one or more of the preceding Claims, **characterized in that** a splayfoot pad (14) is inserted in the region of the foot sole behind the base joints of the toes for retrocapital support of the metatarsus.

13. Device according to one or more of the preceding Claims, **characterized in that** the fixing devices (8a, 8b) are designed as pockets which are sewn onto the stocking (2) or are attached thereto in some other way.

14. Device according to one or more of the preceding Claims, **characterized in that** the stocking (2) is designed as a compression stocking.

15. Device according to one or more of the preceding Claims, **characterized in that** the articulated splint limbs (10, 11) in each case have a free end (15) and an articulation-side end (16), wherein the articulation-side ends (16) have approximately a spherical cap shape and are designed to correspond to one another in such a way that the respective articulation-side ends (16) of the articulated splint limbs (10, 11) can be placed one inside the other in a form-fitting manner.

16. Device according to one or more of the preceding Claims, **characterized in that** the articulated splint limbs (10, 11) and the articulation device (13) are adapted in terms of their three-dimensional shape to the foot contour of the patient.

17. Device according to one or more of the preceding Claims, **characterized in that** the articulation-side ends (16) of the articulated splint limbs (10, 11) have a three-dimensional shape corresponding to one another which is rotationally symmetrical about the axis (12).

18. Device according to one or more of the preceding Claims, **characterized in that** the articulated splint limbs (10, 11) are made of metal or plastic, in particular of a carbon-fibre-reinforced thin plate.

19. Device according to one or more of the preceding Claims, **characterized in that** a force F1 can be exerted on the big toe in the direction of the foot inner side by the flexion splint (9) in order to lateralize the big toe.

20. Device according to one or more of the preceding Claims, **characterized in that** there are means for allowing the force F1 to act also on one or more adjacent toes, wherein the means are tension elements which join toe holders for several toes of a foot to one another.

21. Device according to one or more of the preceding Claims, **characterized in that** the articulated splint limbs (10, 11) have an essentially plate-like three-dimensional shape which is curved in the longitudinal and transverse direction and comprises a first longitudinal boundary edge (52, 82) and a second longitudinal boundary edge (53, 83) and also a narrow boundary edge (54, 84), wherein slot-shaped openings (55, 85) are provided in the region of the longitudinal boundary edges parallel to the course thereof, so that a central web (56, 86), edge webs (57, 87) and intermediate webs (88) are formed.

22. Device according to one or more of the preceding Claims, **characterized in that** the annular bandages (5, 6) are connected to the articulated splint limbs (10, 11).

23. Device according to one or more of the preceding claims, **characterized in that** the annular bandages (5, 6) comprise looped bodies (60, 50) and free ends (51, 61).

24. Device according to one or more of the preceding Claims, **characterized in that** the articulation device (13) is designed as an annular articulation comprising an articulation ring (70) and an articulation disc (90).

25. Device according to one or more of the preceding Claims, **characterized in that** the articulation ring (70) is connected in one piece with the first articulated splint limb (10) and/or the articulation disc (90) is connected in one piece with the second articulated splint limb (11).

26. Device according to one or more of the preceding Claims, **characterized in that** the articulated splint limbs (10, 11) are in each case designed to taper in terms of their material thickness towards their edge regions, and the surface areas of the articulated splint (9) which bear against the foot of the patient are designed to be smooth.

27. Device according to one or more of the preceding Claims, **characterized in that** an annular web (91) is integrally formed on the articulation disc (90), which annular web cooperates with the articulation ring (70) so that a radial guidance of the articulation disc (90) and of the articulation ring (70) with respect to one another is ensured.

28. Device according to one or more of the preceding claims, **characterized in that**, in order to axially fix the articulation ring (70) with respect to the articulation disc (90), latching elements (92) are provided which cooperate with a step, in particular an annular step (73).

29. Device according to one or more of the preceding claims, **characterized in that** the articulation device (13) is covered by a closure device (100), wherein the closure device (100) is connected to the articulated splint (9) by catch means (96, 101) .

30. Device according to one or more of the preceding Claims, **characterized in that** the articulated splint limbs (10, 11), in their longitudinal dimension, enclose an angle a, β with the pivot axis (12), wherein the angles a and β are selected such that the articulated splint (9) can be placed against a patient's foot in such a way that the pivot axis (12) of the articulation device (13) is more or less aligned with the anatomical axis of articulation of the base joint of the big toe.

31. Device according to one or more of the preceding Claims, **characterized in that** the angle a is between 75° and 115°.

32. Device according to one or more of the preceding Claims, **characterized in that** the angle β is approximately 70° to 110°.

33. Device according to one or more of the preceding Claims, **characterized in that** the closure device (100) is designed as a thrust bearing.

34. Device according to one or more of the preceding Claims, **characterized in that** the articulated splint (9) is made of plastic, in particular of impact-resistant and skin-compatible plastic, for example from the polycarbonate group.

35. Device according to one or more of the preceding Claims, **characterized in that**, in being passed or threaded through the articulated splint limbs (10, 11), the bandages (5, 6) are arranged with no steps in the direction of the metatarsus or the big toe of the patient.

36. Device according to one or more of the preceding Claims, **characterized in that** the device (1) is not rigidly attached to the foot so that, when it is being worn, the device (1) can be individually adapted to the anatomical conditions of the patient's foot in terms of the position of the axis of articulation (12) of the articulation device (13).

## Revendications

1. Appareillage orthopédique pour la correction de fausses positions des orteils comportant un premier dispositif de fixation (8a, 6) dans la région du gros orteil, un deuxième dispositif de fixation (8b, 5) dans la région du métatarse et une attelle de flexion (9) qui est maintenue par les dispositifs de fixation (8a, 8b, 5, 11) et qui s'étend le long de la face interne du pied, **caractérisé en ce que** l'attelle de flexion (9) est configurée comme une attelle de flexion articulée qui est configurée de manière articulée dans la direction de flexion-extension (20) de l'orteil ou des orteils à corriger et qui comporte un dispositif articulé (13) qui présente un axe de pivotement (12) qui correspond à peu près à l'axe de pivotement de l'articulation basale du gros orteil dans la direction de flexion-extension.

2. Appareillage selon la revendication 1, **caractérisé en ce que** l'appareillage orthopédique (1) comporte un bas (2) ou un élément enveloppant similaire pour un pied qui porte les dispositifs de fixation (8a, 8b, 5, 6) ainsi que l'attelle de flexion (9).

3. Appareillage selon la revendication 1 et/ou 2, **caractérisé en ce que** l'attelle de flexion (9) comporte des branches d'attelle articulée (10, 11), la section transversale des branches d'attelle articulée (10, 11) ayant une forme lenticulaire.

4. Appareillage selon une ou plusieurs des revendications précédentes, **caractérisé en ce que** le bas (2) est ouvert dans la région des orteils.

5. Appareillage selon une ou plusieurs des revendications précédentes, **caractérisé en ce que** l'appareillage (1) comporte un logement pour gros orteil, par exemple un compartiment pour gros orteil (4), qui est relié en une seule partie au bas (2) ou qui est rajouté à ce bas (2), le compartiment pour gros orteil (4) entourant complètement le pourtour du gros orteil et étant ouvert ou fermé dans la région de l'extrémité libre de l'orteil.

6. Appareillage selon une ou plusieurs des revendications précédentes, **caractérisé en ce que** l'appareillage (1) comporte un premier bandage annulaire (5) dans la région du métatarse, lequel bandage entoure le métatarse, de préférence complètement, et est relié avec le bas (2).

7. Appareillage selon une ou plusieurs des revendications précédentes, **caractérisé en ce qu'**un second bandage annulaire (6) est disposé dans la région de l'extrémité libre du gros orteil, de manière à entourer le compartiment pour gros orteil (4), lequel bandage entoure complètement le pourtour du gros orteil.

8. Appareillage selon une ou plusieurs des revendications précédentes, **caractérisé en ce que** les bandages annulaires (5, 6) sont constitués d'un matériau flexible, souple, circulairement rigide en traction.

9. Appareillage selon une ou plusieurs des revendications précédentes, **caractérisé en ce que**, dans la région d'une face interne du pied (7), le premier bandage annulaire (5) ainsi que le second bandage annulaire (6) ne sont pas reliés avec le bas (2) ou avec le compartiment pour gros orteil (4) sur des tronçons partiels, de sorte que des dispositifs de fixation/de logement (8a, 8b) sont formés entre les bandages annulaires (5, 6) et le compartiment pour gros orteil (4) ou le bas (2).

10. Appareillage selon une ou plusieurs des revendications précédentes, **caractérisé en ce que** les dispositifs de fixation (8a, 8b) sont des goussets emboîtables.

11. Appareillage selon une ou plusieurs des revendications précédentes, **caractérisé en ce que** l'attelle de flexion (9) comporte une première branche d'attelle articulée (10) et une seconde branche d'attelle articulée (11) qui sont reliées de manière articulée à l'aide d'un dispositif articulé (13) de manière à pivoter autour de l'axe de pivotement (12), la première branche d'attelle articulée (10) s'étendant à partir du dispositif articulé (13) jusqu'au dispositif de fixation (8a) et la seconde branche d'attelle articulée (11) s'étendant jusqu'au second dispositif de fixation (8b).

12. Appareillage selon une ou plusieurs des revendications précédentes, **caractérisé en ce qu'**une pelote pour pied plat étalé (14) est insérée dans la région de la plante du pied derrière les articulations basales des orteils, pour l'appui rétrocapital du métatarse.

13. Appareillage selon une ou plusieurs des revendications précédentes, **caractérisé en ce que** les dispositifs de fixation (8a, 8b) sont configurés comme des goussets cousus ou fixés d'une autre manière sur le bas (2).

14. Appareillage selon une ou plusieurs des revendications précédentes, **caractérisé en ce que** le bas (2) est configuré comme un bas de compression.

15. Appareillage selon une ou plusieurs des revendications précédentes, **caractérisé en ce que** les branches d'attelle articulée (10, 11), comportent chacune une extrémité libre (15) et une extrémité du côté de l'articulation (16), les extrémités du côté de l'articulation (16) présentant à peu près une forme tridimensionnelle de calotte sphérique et étant configurées en correspondance réciproque de telle manière que les extrémités respectives du côté de l'articulation (16) des branches d'attelle articulée (10, 11) peuvent être posées l'une dans l'autre pour s'ajuster solidairement.

16. Appareillage selon une ou plusieurs des revendications précédentes, **caractérisé en ce que** la forme tridimensionnelle des branches d'attelle articulée (10, 11) et du dispositif articulé (13) est adaptée au contour du pied du patient.

17. Appareillage selon une ou plusieurs des revendications précédentes, **caractérisé en ce que** les extrémités du côté de l'articulation (16) des branches d'attelle articulée (10, 11) présentent une forme tridimensionnelle en correspondance réciproque, symétrique en rotation autour de l'axe (12).

18. Appareillage selon une ou plusieurs des revendications précédentes, **caractérisé en ce que** les branches d'attelle articulée (10, 11) sont constituées de métal ou de matière synthétique, en particulier d'une plaque mince renforcée par des fibres de carbone.

19. Appareillage selon une ou plusieurs des revendications précédentes, **caractérisé en ce qu'**une force F1 peut être exercée par l'attelle de flexion (9) sur le gros orteil dans la direction de la face interne du pied pour la latéralisation du gros orteil.

20. Appareillage selon une ou plusieurs des revendications précédentes, **caractérisé en ce qu'**il est prévu des dispositifs pour que la force F1 puisse s'exercer également sur un ou plusieurs orteils voisins, les dispositifs étant des éléments de traction qui relient ensemble les logements d'orteil pour plusieurs orteils d'un pied.

21. Appareillage selon une ou plusieurs des revendications précédentes, **caractérisé en ce que** les branches d'attelle articulée (10, 11) présentent une forme tridimensionnelle bombée dans la direction longitudinale et transversale, pour l'essentiel en forme de plaque, avec une première arête de délimitation longitudinale (52, 82) et une seconde arête de délimitation longitudinale (53, 83) ainsi qu'une arête de délimitation étroite (54, 84), des ouvertures en forme de fente (55, 85) étant prévues dans la zone des arêtes de délimitation longitudinale parallèlement à leur tracé, de sorte qu'une nervure médiane (56, 86), des nervures marginales (57, 87) et des nervures intermédiaires (88) sont formées.

22. Appareillage selon une ou plusieurs des revendications précédentes, **caractérisé en ce que** les bandages annulaires (5, 6) sont reliés aux branches d'attelle articulée (10, 11).

23. Appareillage selon une ou plusieurs des revendications précédentes, **caractérisé en ce que** les bandages annulaires (5, 6) comportent des corps à boucle (60, 50) et des extrémités libres (51, 61).

24. Appareillage selon une ou plusieurs des revendications précédentes, **caractérisé en ce que** le dispositif articulé (13) est configuré comme une articulation annulaire avec un anneau articulé (70) et un disque articulé (90)

25. Appareillage selon une ou plusieurs des revendications précédentes, **caractérisé en ce que** l'anneau articulé (70) est relié en une seule pièce à la première branche d'attelle articulée (10) et/ou le disque articulé (90) est relié en une seule pièce à la seconde branche d'attelle articulée (11).

26. Appareillage selon une ou plusieurs des revendications précédentes, **caractérisé en ce que** les branches d'attelle articulée (10, 11) s'amincissent chacune dans l'épaisseur du matériau en direction de leurs zones marginales et les zones de surface de l'attelle articulée (9) adjacentes au pied du patient sont lisses.

27. Appareillage selon une ou plusieurs des revendications précédentes, **caractérisé en ce qu'**une nervure annulaire (91), qui coopère avec l'anneau articulé (70), est moulée sur le disque articulé (90), de sorte qu'un guidage radial du disque articulé (90) et de l'anneau articulé (70) l'un par rapport à l'autre est garanti.

28. Appareillage selon une ou plusieurs des revendications précédentes, **caractérisé en ce que**, pour la fixation axiale de l'anneau articulé (70) par rapport au disque articulé (90), il est prévu des éléments d'encliquetage (92) qui coopèrent avec un redent, en particulier un redent annulaire (73).

29. Appareillage selon une ou plusieurs des revendications précédentes, **caractérisé en ce que** le dispositif articulé (13) est recouvert au moyen d'un dispositif d'obturation (100), le dispositif d'obturation (100) étant relié à l'attelle articulée (9) au moyen de dispositifs d'encliquetage (96, 101).

30. Appareillage selon une ou plusieurs des revendications précédentes, **caractérisé en ce que** les branches d'attelle articulée (10, 11) forment un angle α, β avec l'axe de pivotement (12) sur leur étendue longitudinale, les angles α et β étant choisis de telle manière que l'attelle articulée (9) peut être appliquée sur le pied d'un patient de manière à ce que l'axe de pivotement (12) du dispositif articulé (13) soit aligné à peu près sur l'axe de pivotement anatomique de l'articulation basale du gros orteil.

31. Appareillage selon une ou plusieurs des revendications précédentes, **caractérisé en ce que** l'angle α est compris entre 75° et 115°.

32. Appareillage selon une ou plusieurs des revendications précédentes, **caractérisé en ce que** l'angle β est compris entre 70° et 110°.

33. Appareillage selon une ou plusieurs des revendications précédentes, **caractérisé en ce que** le dispositif d'obturation (100) est configuré comme un palier de butée.

34. Appareillage selon une ou plusieurs des revendications précédentes, **caractérisé en ce que** l'attelle articulée (9) est constituée d'une matière synthétique, en particulier d'une matière synthétique résistante aux chocs et compatible avec la peau, choisie par exemple dans le groupe des polycarbonates.

35. Appareillage selon une ou plusieurs des revendications précédentes, **caractérisé en ce que** les bandages (5, 6) sont disposés en continu en direction du métatarse ou du gros orteil du patient pour leur guidage ou leur enfilement dans les branches d'attelle articulée (10, 11).

36. Appareillage selon une ou plusieurs des revendications précédentes, **caractérisé en ce que** l'appareillage (1) n'est pas attaché rigidement au pied, de sorte que, lorsqu'il est porté, l'appareillage (1) peut s'adapter individuellement aux conditions marginales du pied du patient en fonction de la position de l'axe de pivotement (12) du dispositif articulé (13).
